# EUROPEAN PATENT APPLICATION

(11) **EP 4 682 158 A1**
(43) Date of publication of application: **21.01.2026**
(21) Application number: 24774846.0
(22) Date of filing: 15.03.2024
(51) Int. Cl.: C07K 7/00, C07K 7/56, C12N 5/071

(54) **CYCLIC PEPTIDE OR SALT THEREOF AND USE THEREOF**

(30) Priority: 17.03.2023 JP 2023043077; 29.09.2023 JP 2023170566
(71) Applicant: FUJIFILM Corporation, Tokyo 106-8620 (JP)
(72) Inventor: IZUTA, Shin, Ashigarakami-gun, Kanagawa 258-8577 (JP); HABA, Ryota, Ashigarakami-gun, Kanagawa 258-8577 (JP); WADA, Shiori, Ashigarakami-gun, Kanagawa 258-8577 (JP); YOSHIMURA, Naoko, Ashigarakami-gun, Kanagawa 258-8577 (JP); TOMINAGA, Jo, Ashigarakami-gun, Kanagawa 258-8577 (JP); ISHII, Yukiko, Ashigarakami-gun, Kanagawa 258-8577 (JP)
(74) Representative: Hoffmann Eitle
(86) International application number: PCT/JP2024/010164
(87) International publication number: WO 2024/195710

(57) **Abstract**

An object of the present invention is to provide a cyclic peptide or a salt thereof and a cyclic peptide complex or a salt thereof, which have excellent binding affinity to an FGFR protein, and to provide a culture medium composition, a material for purification, a material for labeling, a material for cell regulation, and a material for accumulation, which use the cyclic peptide or a salt thereof and the cyclic peptide complex or a salt thereof.

According to the present invention, there is provided a cyclic peptide or a salt thereof, the cyclic peptide being a peptide having an amino acid sequence represented by X1-Xm-X2-Xn-X3, in which the cyclic peptide contains a cyclized portion that is cyclized via covalent bonding, and the cyclized portion includes a structure represented by Formula (2),

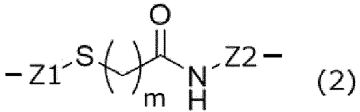

X1 represents a glutamine residue or a histidine residue, Xm represents a peptide residue containing any 2 to 4 amino acid residues, X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue, Xn represents an amino acid residue or a peptide residue containing any 1 to 3 amino acid residues, and X3 represents an amino acid residue containing an aromatic residue on a side chain, in Formula (2), Z1 and Z2 each independently represent a linking group, and m represents an integer of 1 to 10.

## Description

### Technical Field

The present invention relates to a cyclic peptide having excellent binding affinity to a fibroblast growth factor receptor (FGFR) protein and excellent stability, or a salt thereof. The present invention further relates to a cyclic peptide complex or a salt thereof, in which two or more molecules of the cyclic peptide or a salt thereof are linked by a linker. The present invention further relates to the use of the cyclic peptide or a salt thereof and the cyclic peptide complex or a salt thereof.

### Background Art

As a treatment method for diseases that have not been treatable with conventional low-molecular-weight drugs and antibody drugs, the development of cell therapy products for administering cells having a healing function into the body is being promoted. Furthermore, in recent years, research and development for practical application of cell products such as cultured meat (cellular agriculture) and cells for drug discovery support, which are expected to be effective against food shortages and greenhouse gases, has been promoted. In the research and manufacture of these cell therapy products and cell products, it is important to efficiently proliferate cells while maintaining the quality such as the undifferentiated potency. Among the components of the culture medium used for cell proliferation, a protein group called a growth factor (also referred to as a cytokine) is an important component that greatly affects the quality and the cell proliferation rate. However, the growth factor is generally very expensive and has low stability, which causes high manufacturing costs of cell therapy products and cell products. In particular, the production of cultured meat is costly and difficult to produce commercially, and a very important cost factor is the necessity of a large amount of special cell culture media, and in particular, a proper growth factor that tends to be the most expensive component of the cell culture medium. Currently, most of the production cost (maximum 96% or more) is affected by the cost of the growth factor, and production is economically impossible.

Among the growth factors, fibroblast growth factor (FGF) is known to play an important role in maintaining the undifferentiated potency of a wide range of cells and promoting cell proliferation. Among these, basic fibroblast growth factor (basic FGF, abbreviation: bFGF) is widely used for the proliferation of mesenchymal stem cells which account for a large number of cell therapy products, and is widely used as a component that is extremely important for maintaining the undifferentiated potency and promoting the proliferation of induced pluripotent stem cells (iPSC) or somatic stem cells.

The maintenance of the undifferentiated potency and the promotion of the proliferation of bFGF are expressed starting from the binding of bFGF to the extracellular region of the fibroblast growth factor receptor (FGFR) protein. Therefore, to reduce the price of bFGF and improve stability thereof, a substance that binds to the extracellular region of the FGFR protein has been studied for a long time.

Patent Document 1 describes a disulfide-type cyclic peptide that binds to an FGFR protein. Patent Document 2 describes a thioether-type cyclic peptide as a substance that achieves both binding affinity and stability in a protein other than FGFR.

### Prior Art Documents

### Patent Documents

Patent Document 1: WO2000/003245A
Patent Document 2: JP2017-95443A

### Summary of Invention

### Object to be solved by the invention

The peptide described in Patent Document 1 has a significantly low binding affinity to the FGFR protein, and thus has no practicality. In addition, it is known that the disulfide bond contained in the disulfide-type cyclic peptide is unstable and is easily decomposed in a cell culture environment or the like. In addition, in a case where the amino acid sequence that binds to FGFR in Patent Document 1 is combined with the stable thioether bond in Patent Document 2, the binding affinity to the FGFR protein is significantly low, and thus there is no practicality.

An object to be achieved of the present invention is to provide a cyclic peptide or a salt thereof and a cyclic peptide complex or a salt thereof, which have excellent binding affinity to an FGFR protein. Another object of the present invention is to provide a culture medium composition, a culture medium additive, a material for purification, a material for labeling, a material for cell regulation, and a material for accumulation, which use the cyclic peptide or a salt thereof and the cyclic peptide complex or a salt thereof.

### Means for solving the object

As a result of intensive studies to achieve the above object, the present inventors have found that a cyclic peptide having a specific amino acid residue at a specific position in an amino acid sequence has excellent binding affinity to an FGFR protein. The present invention has been completed based on the above findings. According to the present invention, the following inventions are provided.
<1> A cyclic peptide or a salt thereof, the cyclic peptide being a peptide having an amino acid sequence represented by X1-Xm-X2-Xn-X3, in which the cyclic peptide contains a cyclized portion that is cyclized via covalent bonding, and the cyclized portion includes a structure represented by Formula (2), 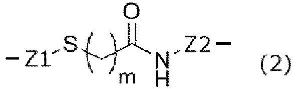
   X1 represents a glutamine residue or a histidine residue,
   Xm represents a peptide residue containing any 2 to 4 amino acid residues,
   X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
   Xn represents an amino acid residue or a peptide residue containing any 1 to 3 amino acid residues, and
   X3 represents an amino acid residue containing an aromatic residue on a side chain, in Formula (2),
   Z1 and Z2 each independently represent a linking group, and
   m represents an integer of 1 to 10.
<2> The cyclic peptide or a salt thereof according to <1>, in which the cyclic peptide is represented by Formula (1-1) or Formula (1-2), 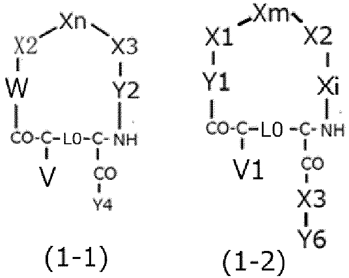 in the formulae,
   W represents -Xm-X1-Y1- or Xk, where Xm is bonded to X2 and Y1 is bonded to CO,
   in a case where W represents -Xm-X1-Y1-, V represents -NH-Y3 and L0 represents L, or V represents R and L0 represents L1,
   in a case where W represents Xk, V represents -NH-X1-Y5 and L0 represents L,
   V1 represents -NH-Y3 or R,
   in a case where V1 represents -NH-Y3, L0 represents L, and in a case where V1 represents R, L0 represents L1,
   X1 represents a glutamine residue or a histidine residue,
   Xm represents a peptide residue containing any 2 to 4 amino acid residues,
   Xk represents a peptide residue containing any 1 to 3 amino acid residues,
   X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
   Xn represents an amino acid residue or a peptide residue containing any 1 to 3 amino acid residues,
   Xi represents a single bond, or an amino acid residue or a peptide residue containing any 1 or 2 amino acid residues,
   X3 represents an amino acid residue containing an aromatic residue on a side chain,
   Y1 represents a single bond, or an amino acid residue or a peptide residue containing any 1 to 10 amino acid residues,
   Y2 represents a single bond, or an amino acid residue or a peptide residue containing any 1 to 12 amino acid residues,
   Y3 represents a hydrogen atom, or an amino acid residue or a peptide residue containing any 1 to 7 amino acid residues,
   Y4 represents OH, or an amino acid residue or a peptide residue containing any 1 to 7 amino acid residues,
   Y5 represents a hydrogen atom, or an amino acid residue or a peptide residue containing any 1 to 7 amino acid residues,
   Y6 represents OH, or an amino acid residue or a peptide residue containing any 1 to 7 amino acid residues,
   L and L1 represent a cyclized portion,
   R represents a side chain structure of an amino acid, and
   an amino terminal of X1 is bonded to a carboxyl terminal of Y1, and a carboxyl terminal of X1 is bonded to an amino terminal of Xm.
<3> The cyclic peptide or a salt thereof according to <1>, in which the cyclic peptide is represented by Formula (1A), Formula (1B), or Formula (1C), 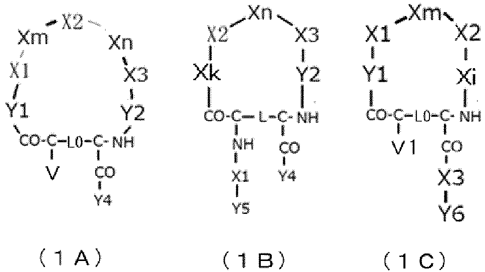 in the formulae,
   X1 represents a glutamine residue or a histidine residue,
   Xm represents a peptide residue containing any 2 to 4 amino acid residues,
   Xk represents a peptide residue containing any 1 to 3 amino acid residues,
   X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
   Xn represents an amino acid residue or a peptide residue containing any 1 to 3 amino acid residues,
   Xi represents a single bond, or an amino acid residue or a peptide residue containing any 1 or 2 amino acid residues,
   X3 represents an amino acid residue containing an aromatic residue on a side chain,
   Y1 represents a single bond, or an amino acid residue or a peptide residue containing any 1 to 10 amino acid residues,
   Y2 represents a single bond, or an amino acid residue or a peptide residue containing any 1 to 12 amino acid residues,
   Y3 represents a hydrogen atom, or an amino acid residue or a peptide residue containing any 1 to 7 amino acid residues,
   Y4 represents OH, or an amino acid residue or a peptide residue containing any 1 to 7 amino acid residues,
   Y5 represents a hydrogen atom, or an amino acid residue or a peptide residue containing any 1 to 7 amino acid residues,
   Y6 represents OH, or an amino acid residue or a peptide residue containing any 1 to 7 amino acid residues,
   V represents -NH-Y3 or R, in a case where V is -NH-Y3, L0 represents L, and in a case where V is R, L0 represents L1,
   V1 represents -NH-Y3 or R, in a case where V1 is -NH-Y3, L0 represents L, and in a case where V1 is R, L0 represents L1,
   L and L1 represent a cyclized portion,
   R represents a side chain structure of an amino acid, and
   an amino terminal of X1 is bonded to a carboxyl terminal of Y1, and a carboxyl terminal of X1 is bonded to an amino terminal of Xm.
<4> The cyclic peptide or a salt thereof according to <1>, in which the cyclic peptide is represented by Formula (1A1), Formula (1A2), Formula (1B), Formula (1C1), or Formula (1C2), 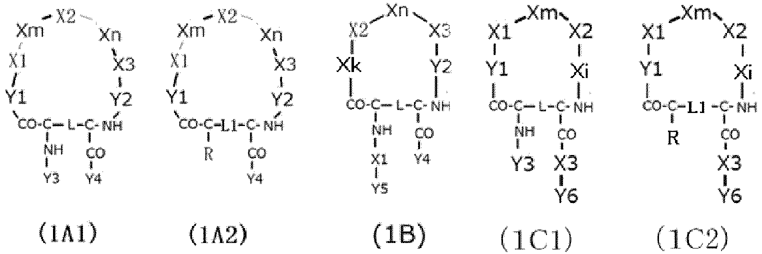 in the formulae,
   X1 represents a glutamine residue or a histidine residue,
   Xm represents a peptide residue containing any 2 to 4 amino acid residues,
   Xk represents a peptide residue containing any 1 to 3 amino acid residues,
   X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
   Xn represents an amino acid residue or a peptide residue containing any 1 to 3 amino acid residues,
   Xi represents a single bond, or an amino acid residue or a peptide residue containing any 1 or 2 amino acid residues,
   X3 represents an amino acid residue containing an aromatic residue on a side chain,
   Y1 represents a single bond, or an amino acid residue or a peptide residue containing any 1 to 10 amino acid residues,
   Y2 represents a single bond, or an amino acid residue or a peptide residue containing any 1 to 12 amino acid residues,
   Y3 represents a hydrogen atom, or an amino acid residue or a peptide residue containing any 1 to 7 amino acid residues,
   Y4 represents OH, or an amino acid residue or a peptide residue containing any 1 to 7 amino acid residues,
   Y5 represents a hydrogen atom, or an amino acid residue or a peptide residue containing any 1 to 7 amino acid residues,
   Y6 represents OH, or an amino acid residue or a peptide residue containing any 1 to 7 amino acid residues,
   L and L1 represent a cyclized portion,
   R represents a side chain structure of an amino acid, and
   an amino terminal of X1 is bonded to a carboxyl terminal of Y1, and a carboxyl terminal of X1 is bonded to an amino terminal of Xm.
<5> The cyclic peptide or a salt thereof according to <1>, in which Xm represents a group represented by -Xo-X4-,
   Xo represents an amino acid residue or a peptide residue containing any 1 to 3 amino acid residues, and
   X4 represents an alanine residue, an isoleucine residue, a phenylalanine residue, a tyrosine residue, an asparagine residue, a histidine residue, or a methionine residue.
<6> The cyclic peptide or a salt thereof according to <1>, in which Xm represents a group represented by -Xo-X4-,
   Xo represents an amino acid residue or a peptide residue containing any 1 to 3 amino acid residues,
   X4 represents an isoleucine residue, a phenylalanine residue, a tyrosine residue, an asparagine residue, a histidine residue, or a methionine residue,
   Xk represents a group represented by -Xj-X4-, and
   Xj represents a single bond, or an amino acid residue or a peptide residue containing any 1 or 2 amino acid residues.
<7> The cyclic peptide or a salt thereof according to <1>, in which Xm represents a group represented by -X5-X6-X4-,
   X5 represents an alanine residue, a proline residue, a leucine residue, an isoleucine residue, a valine residue, a phenylalanine residue, a tyrosine residue, a tryptophan residue, an asparagine residue, a serine residue, a glutamine residue, a threonine residue, an aspartic acid residue, a glutamic acid residue, an arginine residue, a histidine residue, a methionine residue, a thiol group-containing amino acid residue forming a cyclized portion, or a halocarboxyl group-containing amino acid residue forming a cyclized portion,
   X6 represents any amino acid residue, and
   X4 represents an alanine residue, an isoleucine residue, a phenylalanine residue, a tyrosine residue, an asparagine residue, a histidine residue, or a methionine residue.
<8> The cyclic peptide or a salt thereof according to <1>, in which Xm represents a group represented by -X5-X6-X4-,
   X5 represents an alanine residue, a proline residue, a leucine residue, an isoleucine residue, a phenylalanine residue, a tyrosine residue, a tryptophan residue, an asparagine residue, a glutamine residue, a threonine residue, an aspartic acid residue, a glutamic acid residue, an arginine residue, a histidine residue, or a methionine residue,
   X6 represents any amino acid residue,
   X4 represents an isoleucine residue, a phenylalanine residue, a tyrosine residue, an asparagine residue, a histidine residue, or a methionine residue, and
   Xk represents a group represented by -X6-X4-.
<9> The cyclic peptide or a salt thereof according to <4>, in which L represents a cyclized portion represented by 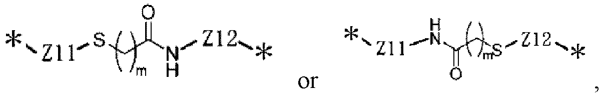 where Z11 and Z12 each independently represent a linking group, m represents an integer of 1 to 10, and * represents a position of an α-carbon atom of an amino acid main chain, and
   L1 represents a cyclized portion represented by 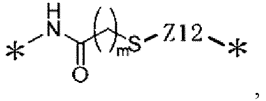 where Z12 represents a linking group, m represents an integer of 1 to 10, and * represents a position of an α-carbon atom of an amino acid main chain.
<10> The cyclic peptide or a salt thereof according to <1>, in which a ring is composed of 10 to 22 amino acid residues.
<11> The cyclic peptide or a salt thereof according to <1>, in which the cyclized portion contains a homocysteine residue.
<12> The cyclic peptide or a salt thereof according to <1>, in which the cyclic peptide has any one of amino acid sequences set forth in SEQ ID NOs: 37 to 450 and 452 to 454 described in Tables 4 to 6, or
   the cyclic peptide has an amino acid sequence in which 1 to 4 amino acids in any one of the amino acid sequences set forth in SEQ ID NOs: 37 to 450 and 452 to 454 described in Tables 4 to 6 have been substituted, deleted, or inserted, and has a binding ability to an FGFR protein.
<13> The cyclic peptide or a salt thereof according to <1>, in which the cyclic peptide has any one of amino acid sequences set forth in SEQ ID NOs: 37 to 51, 53 to 55, 57 to 61, 63 to 68, 70 to 89, 92, 93, 103, 104, 106 to 109, 114, 115, 117, 119, 121 to 123, 125 to 133, 136 to 181, 187 to 189, 194 to 199, 201 to 203, 205 to 207, 211 to 218, 220 to 235, 237 to 250, 254 to 270, 272, 275, 276, 278 to 291, 293 to 324, 328 to 337, 339, 341 to 354, 356, 358 to 360, 385, 386, 391, 392, 394 to 398, 400, 402, 403, 406, 407, 409 to 411, 430, 433 to 435, 437 to 444, 446 to 450, and 452 described in Tables 4 to 6, or
   the cyclic peptide has an amino acid sequence in which 1 to 4 amino acids in any one of the amino acid sequences set forth in SEQ ID NOs: 37 to 51, 53 to 55, 57 to 61, 63 to 68, 70 to 89, 92, 93, 103, 104, 106 to 109, 114, 115, 117, 119, 121 to 123, 125 to 133, 136 to 181, 187 to 189, 194 to 199, 201 to 203, 205 to 207, 211 to 218, 220 to 235, 237 to 250, 254 to 270, 272, 275, 276, 278 to 291, 293 to 324, 328 to 337, 339, 341 to 354, 356, 358 to 360, 385, 386, 391, 392, 394 to 398, 400, 402, 403, 406, 407, 409 to 411, 430, 433 to 435, 437 to 444, 446 to 450, and 452 described in Tables 4 to 6 have been substituted, deleted, or inserted, and has a binding ability to an FGFR protein.
<14> The cyclic peptide or a salt thereof according to <1>, in which the cyclic peptide has any one of amino acid sequences set forth in SEQ ID NOs: 37 to 42, 45, 47 to 51, 53 to 55, 60 to 61, 63, 65 to 68, 71 to 88, 92 to 93, 106, 108, 114 to 115, 117, 119, 121, 123, 125 to 133, 136 to 170, 172 to 180, 187 to 188, 194 to 199, 201, 203, 205 to 207, 211 to 212, 214 to 218, 220 to 235, 237 to 250, 254 to 260, 262 to 270, 272, 275, 278 to 290, 293, 295 to 324, 328 to 337, 339, 341 to 354, 356, 358 to 360, 391 to 392, 395, 397, 400, 402, 406, 409 to 411, 430, 433 to 435, 437 to 443, 446 to 450, and 452 described in Tables 4 to 6, or
   the cyclic peptide has an amino acid sequence in which 1 to 4 amino acids in any one of the amino acid sequences set forth in SEQ ID NOs: 37 to 42, 45, 47 to 51, 53 to 55, 60 to 61, 63, 65 to 68, 71 to 88, 92 to 93, 106, 108, 114 to 115, 117, 119, 121, 123, 125 to 133, 136 to 170, 172 to 180, 187 to 188, 194 to 199, 201, 203, 205 to 207, 211 to 212, 214 to 218, 220 to 235, 237 to 250, 254 to 260, 262 to 270, 272, 275, 278 to 290, 293, 295 to 324, 328 to 337, 339, 341 to 354, 356, 358 to 360, 391 to 392, 395, 397, 400, 402, 406, 409 to 411, 430, 433 to 435, 437 to 443, 446 to 450, and 452 described in Tables 4 to 6 have been substituted, deleted, or inserted, and has a binding ability to an FGFR protein.
<15> The cyclic peptide or a salt thereof according to <1>, in which the cyclic peptide is modified with another substance.
<16> A cyclic peptide complex or a salt thereof, in which two or more molecules of the cyclic peptide or a salt thereof according to any one of <1> to <15> are linked by a linker.
<17> A cyclic peptide complex or a salt thereof, in which two molecules of the cyclic peptide or a salt thereof according to any one of <1> to <15> are linked by a linker.
<18> The cyclic peptide complex or salt thereof according to <16>, in which the cyclic peptide complex is modified with another substance.
<19> A culture medium composition or a culture medium additive, comprising:
   the cyclic peptide or a salt thereof according to any one of <1> to <15>.
<20> A material for purification comprising:
   the cyclic peptide or a salt thereof according to any one of <1> to <15>.
<21> A material for labeling comprising:
   the cyclic peptide or a salt thereof according to any one of <1> to <15>.
<22> A material for cell regulation comprising:
   the cyclic peptide or a salt thereof according to any one of <1> to <15>.
<23> A material for accumulation comprising:
   the cyclic peptide or a salt thereof according to any one of <1> to <15>.
<24> A culture medium composition or a culture medium additive, comprising:
   the cyclic peptide complex or a salt thereof according to <16>.
<25> A material for purification comprising:
   the cyclic peptide complex or a salt thereof according to <16>.
<26> A material for labeling comprising:
   the cyclic peptide complex or a salt thereof according to <16>.
<27> A material for cell regulation comprising:
   the cyclic peptide complex or a salt thereof according to <16>.
<28> A material for accumulation comprising:
   the cyclic peptide complex or a salt thereof according to <16>.

### Effect of the invention

The cyclic peptide or a salt thereof according to the present invention has excellent binding affinity to an FGFR protein.

### BRIEF DESCRIPTION OF THE DRAWINGS

[Fig. 1] Fig. 1 shows structures of compounds used in Examples.
[Fig. 2] Fig. 2 shows the cell proliferation-promoting effect of a cyclic peptide complex (SEQ ID NO: 458).
[Fig. 3] Fig. 3 shows the bovine cell proliferation effect of the cyclic peptide complex (SEQ ID NO: 458).

### Embodiments for carrying out the invention

Hereinafter, the present invention is specifically described. These descriptions and Examples are only illustrative of the embodiments and do not limit the ranges of the embodiments. The action mechanism mentioned in the present disclosure includes estimation, and the accuracy thereof does not limit the ranges of the embodiments.

The term "step" or a term representing a step in the present disclosure not only includes, in addition to a step independent of another step, a step that achieves a desired effect of the step even in a case of not being clearly distinguished from the other step.

In the present disclosure, a numerical range expressed using "to" indicates a range including numerical values before and after "to" as a minimum value and a maximum value.

In the numerical ranges described stepwise in the present disclosure, the upper limit value or the lower limit value described in one numerical range may be replaced with the upper limit value or the lower limit value of the numerical range described stepwise in other stages. In addition, in the numerical ranges described in the present disclosure, the upper limit value or the lower limit value of the numerical range may be replaced with the value shown in Examples.

### <Amino acid, amino acid residue, and peptide>

The amino acids are generally represented by names, abbreviations, and the like adopted by the International Union of Pure and Applied Chemistry and International Union of Biochemistry and Molecular Biology (IUPAC-IUB Joint Commission on Biochemical Nomenclature (JCBN)). In addition, the amino acid residue is represented by an abbreviation of the amino acid from which the amino acid residue is derived. It is noted that the amino acid residue may be an N-terminal amino acid (N-terminal residue) or a C-terminal amino acid (C-terminal residue).

Unless otherwise specified, an amino acid sequence of a peptide or a protein (also referred to as a "primary structure") is represented by arranging amino acid residues in a linear sequence from the N-terminal at the left end to the C-terminal at the right end.

Examples of the amino acid include an α-amino acid, a β-amino acid, a γ-amino acid, and an amino acid in which an amino group or a carboxyl group is substituted with a similarly reactive group (for example, a primary amine is substituted with a secondary or tertiary amine, or a carboxyl group is substituted with an ester), but the amino acid is not particularly limited. The α-amino acid refers to a molecule containing an amino group and a carboxyl group which are bonded to a carbon designated as an α-carbon. The β-amino acid refers to a molecule containing both an amino group and a carboxyl group in a β configuration. The γ-amino acid refers to a molecule containing both an amino group and a carboxyl group in a γ configuration. The amino acid is preferably an α-amino acid.

The amino acid may be any of a natural amino acid or an unnatural amino acid and may be any of D- or L-isomers. However, in a case where an amino acid is represented by its name and isomers having an enantiomeric relationship, that is, an L-form and a D-form are present, the amino acid is represented by an L-form in principle, unless the L-form and the D-form are explicitly distinguished. For example, "isoleucine" represents "L-isoleucine", and an enantiomer of "isoleucine" represents "D-isoleucine". The same applies to the amino acid residues.

Table 1 shows the names and abbreviations (one-letter abbreviations and three-letter abbreviations) of amino acids officially recognized for one-letter abbreviations and three-letter abbreviations.

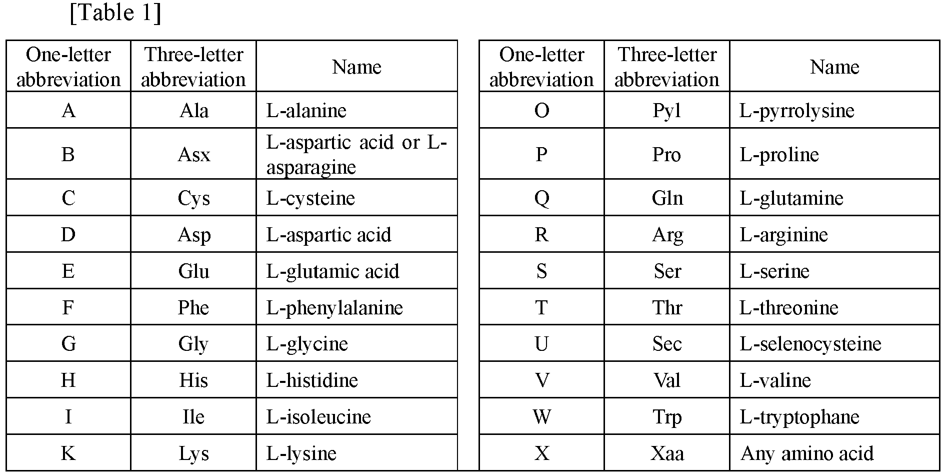

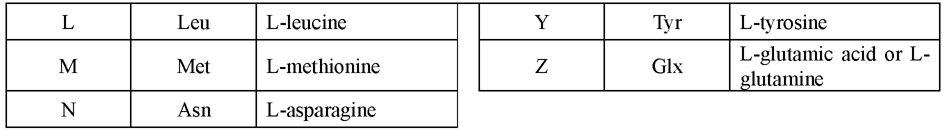

The amino acid is not limited to those listed in Table 1, and an amino acid referred to as an unnatural amino acid can also be used. Examples of the unnatural amino acid are shown in Table 2 below, but the examples are not limited thereto. In addition, the amino acid may be an N-alkyl amino acid in which a hydrogen atom on an amino group at an N-terminal is substituted with an alkyl group (a methyl group, a propyl group, or the like). Examples of the N-alkyl amino acid include N-methyl leucine (hereinafter, referred to as ^{me}L).

**[Table 2]**

| Three-letter abbreviation | Name | | Three-letter abbreviation | Name |
|---|---|---|---|---|
| Aad | Homoglutamic acid | | Gla | 4-Carboxygluamic acid |
| βAad | 3-Aminoadipic acid | | Glp | 5-Oxoproline |
| Abu | 2-Aminobutanoic acid | | Hcy | Homocysteine |
| Dab | 2,4-Diaminobutanoic acid | | Hse | Homoserine |
| Ahx | 2-Aminohexanoic acid | | Hsl | Homoserine lactone |
| Ahe | 2-Aminoheptanoic acid | | 5Hyl | 5-Hydroxylysine (Hyl) |
| Aib | 2-Aminoisobutyric acid | | aHyl | Allo-hydroxylysine |
| εAhx | 6-Aminohexanoic acid | | 3Hyp | 3-Hydroxyproline |
| βAla | β-alanine | | 4Hyp | 4-Hydroxyproline |
| Ape | 2-Aminopentanoic acid | | Hph | Homophenylalanine |
| A₂pr | 2,3-Diaminopropanoic acid | | Hle | Homoleucine |
| Apm | 2-Aminopimelic acid | | Hty | Homotyrosine |
| A₂pm | 2,6-Diaminopimelic acid | | aIle | Allo-isoleucine |
| Cit | Citrulline | | Nle | Norleucine |
| Cya | Cysteic acid | | Nva | Norvaline |
| Dbu | 2,4-Diaminobutanoic acid | | Orn | Ornithine |
| Dpm | 2,6-Diaminopimelic acid | | Sar | Sarcosine |
| Dap | 2,3-Diaminopropanoic acid | | aThr | Allo-threonine |
| Pen | Penicillamine | | Thx | Thyroxine |

Examples of the amino acid containing an aromatic residue include phenylalanine, tryptophan, tyrosine, histidine, and an unnatural amino acid containing a benzene ring, an imidazole ring, or a pyridine ring in a side chain structure, and phenylalanine, tryptophan, and tyrosine are preferable.

As the amino acid, methionine, cysteine, and lysine may be used, but from the viewpoint of oxidation resistance, it is preferable not use methionine and cysteine, and from the viewpoint of the cost of chemical synthesis of peptides, it is preferable not to include cysteine and lysine.

The peptide generally refers to a structure in which 3 to 100 amino acids are linked, but a structure other than the amino acid may be included at a terminal and/or inside the structure. All substances in which 3 to 100 amino acids are included are peptides.

The cyclic peptide refers to a peptide that encompasses a closed ring structure composed of three or more amino acid residues.

### <Cyclic peptide>

The cyclic peptide according to the embodiment of the present invention is a peptide having an amino acid sequence represented by X1-Xm-X2-Xn-X3, in which the cyclic peptide contains a cyclized portion that is cyclized via covalent bonding, and the cyclized portion includes a structure represented by Formula (2), 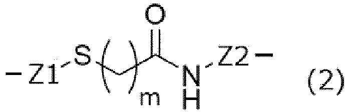
X1 represents a glutamine residue or a histidine residue,
Xm represents a peptide residue containing any 2 to 4 amino acid residues,
X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
Xn represents an amino acid residue or a peptide residue containing any 1 to 3 amino acid residues, and
X3 represents an amino acid residue containing an aromatic residue on a side chain, in Formula (2),
Z1 and Z2 each independently represent a linking group, and
m represents an integer of 1 to 10.

Preferably, the cyclic peptide according to the embodiment of the present invention is represented by Formula (1-1) or Formula (1-2). 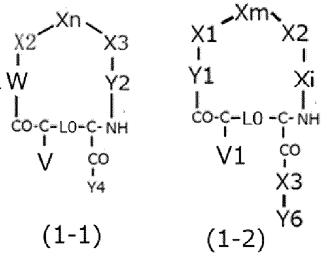 In the formulae,
W represents -Xm-X1-Y1- or Xk, where Xm is bonded to X2 and Y1 is bonded to CO,
in a case where W represents -Xm-X1-Y1-, V represents -NH-Y3 and L0 represents L, or V represents R and L0 represents L1,
in a case where W represents Xk, V represents -NH-X1-Y5 and L0 represents L,
V1 represents -NH-Y3 or R,
in a case where V1 represents -NH-Y3, L0 represents L, and in a case where V1 represents R, L0 represents L1,
X1 represents a glutamine residue or a histidine residue,
Xm represents a peptide residue containing any 2 to 4 amino acid residues,
Xk represents a peptide residue containing any 1 to 3 amino acid residues,
X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
Xn represents an amino acid residue or a peptide residue containing any 1 to 3 amino acid residues,
Xi represents a single bond, or an amino acid residue or a peptide residue containing any 1 or 2 amino acid residues,
X3 represents an amino acid residue containing an aromatic residue on a side chain,
Y1 represents a single bond, or an amino acid residue or a peptide residue containing any 1 to 10 amino acid residues,
Y2 represents a single bond, or an amino acid residue or a peptide residue containing any 1 to 12 amino acid residues,
Y3 represents a hydrogen atom, or an amino acid residue or a peptide residue containing any 1 to 7 amino acid residues,
Y4 represents OH, or an amino acid residue or a peptide residue containing any 1 to 7 amino acid residues,
Y5 represents a hydrogen atom, or an amino acid residue or a peptide residue containing any 1 to 7 amino acid residues,
Y6 represents OH, or an amino acid residue or a peptide residue containing any 1 to 7 amino acid residues,
L and L1 represent a cyclized portion,
R represents a side chain structure of an amino acid, and
an amino terminal of X1 is bonded to a carboxyl terminal of Y1, and a carboxyl terminal of X1 is bonded to an amino terminal of Xm.

Preferably, the cyclic peptide according to the embodiment of the present invention is represented by Formula (1A), Formula (1B), or Formula (1C). 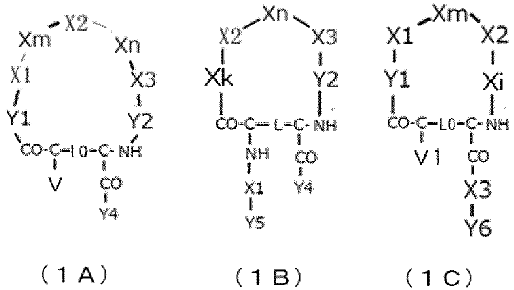 **In** the formulae,
X1 represents a glutamine residue or a histidine residue,
Xm represents a peptide residue containing any 2 to 4 amino acid residues,
Xk represents a peptide residue containing any 1 to 3 amino acid residues,
X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
Xn represents an amino acid residue or a peptide residue containing any 1 to 3 amino acid residues,
Xi represents a single bond, or an amino acid residue or a peptide residue containing any 1 or 2 amino acid residues,
X3 represents an amino acid residue containing an aromatic residue on a side chain,
Y1 represents a single bond, or an amino acid residue or a peptide residue containing any 1 to 10 amino acid residues,
Y2 represents a single bond, or an amino acid residue or a peptide residue containing any 1 to 12 amino acid residues,
Y3 represents a hydrogen atom, or an amino acid residue or a peptide residue containing any 1 to 7 amino acid residues,
Y4 represents OH, or an amino acid residue or a peptide residue containing any 1 to 7 amino acid residues,
Y5 represents a hydrogen atom, or an amino acid residue or a peptide residue containing any 1 to 7 amino acid residues,
Y6 represents OH, or an amino acid residue or a peptide residue containing any 1 to 7 amino acid residues,
V represents -NH-Y3 or R, in a case where V is -NH-Y3, L0 represents L, and in a case where V is R, L0 represents L1,
V1 represents -NH-Y3 or R, in a case where V1 is -NH-Y3, L0 represents L, and in a case where V1 is R, L0 represents L1,
L and L1 represent a cyclized portion,
R represents a side chain structure of an amino acid, and
an amino terminal of X1 is bonded to a carboxyl terminal of Y1, and a carboxyl terminal of X1 is bonded to an amino terminal of Xm.

Preferably, the cyclic peptide according to the embodiment of the present invention is represented by Formula (1A1), Formula (1A2), Formula (1B), Formula (1C1), or Formula (1C2). 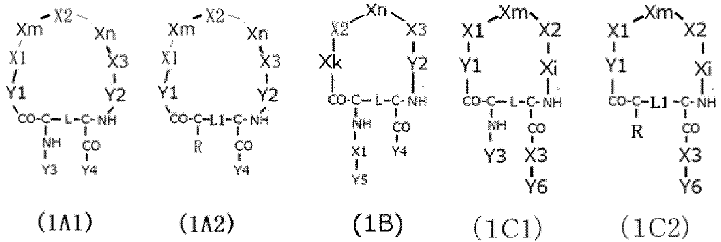 In the formulae,
X1 represents a glutamine residue or a histidine residue,
Xm represents a peptide residue containing any 2 to 4 amino acid residues,
Xk represents a peptide residue containing any 1 to 3 amino acid residues,
X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
Xn represents an amino acid residue or a peptide residue containing any 1 to 3 amino acid residues,
Xi represents a single bond, or an amino acid residue or a peptide residue containing any 1 or 2 amino acid residues,
X3 represents an amino acid residue containing an aromatic residue on a side chain,
Y1 represents a single bond, or an amino acid residue or a peptide residue containing any 1 to 10 amino acid residues,
Y2 represents a single bond, or an amino acid residue or a peptide residue containing any 1 to 12 amino acid residues,
Y3 represents a hydrogen atom, or an amino acid residue or a peptide residue containing any 1 to 7 amino acid residues,
Y4 represents OH, or an amino acid residue or a peptide residue containing any 1 to 7 amino acid residues,
Y5 represents a hydrogen atom, or an amino acid residue or a peptide residue containing any 1 to 7 amino acid residues,
Y6 represents OH, or an amino acid residue or a peptide residue containing any 1 to 7 amino acid residues,
L and L1 represent a cyclized portion,
R represents a side chain structure of an amino acid, and
an amino terminal of X1 is bonded to a carboxyl terminal of Y1, and a carboxyl terminal of X1 is bonded to an amino terminal of Xm.

In a more preferred aspect,
Xm represents a group represented by -Xo-X4-,
Xo represents an amino acid residue or a peptide residue containing any 1 to 3 amino acid residues,
X4 represents an alanine residue, an isoleucine residue, a phenylalanine residue, a tyrosine residue, an asparagine residue, a histidine residue, or a methionine residue,
Xk represents a group represented by -Xj-X4-, and
Xj represents a single bond, or an amino acid residue or a peptide residue containing any 1 or 2 amino acid residues,
**In** a more preferred aspect,
Xm represents a group represented by -Xo-X4-,
Xo represents an amino acid residue or a peptide residue containing any 1 to 3 amino acid residues,
X4 represents an isoleucine residue, a phenylalanine residue, a tyrosine residue, an asparagine residue, a histidine residue, or a methionine residue,
Xk represents a group represented by -Xj-X4-, and
Xj represents a single bond, or an amino acid residue or a peptide residue containing any 1 or 2 amino acid residues.

In a still more preferred aspect,
Xm represents a group represented by -X5-X6-X4-,
X5 represents an alanine residue, a proline residue, a leucine residue, an isoleucine residue, a valine residue, a phenylalanine residue, a tyrosine residue, a tryptophan residue, an asparagine residue, a serine residue, a glutamine residue, a threonine residue, an aspartic acid residue, a glutamic acid residue, an arginine residue, a histidine residue, a methionine residue, a thiol group-containing amino acid residue forming a cyclized portion, or a halocarboxyl group-containing amino acid residue forming a cyclized portion,
X6 represents any amino acid residue,
X4 represents an alanine residue, an isoleucine residue, a phenylalanine residue, a tyrosine residue, an asparagine residue, a histidine residue, or a methionine residue, and
Xk represents a group represented by -X6-X4-.

In a still more preferred aspect,
Xm represents a group represented by -X5-X6-X4-,
X5 represents an alanine residue, a proline residue, a leucine residue, an isoleucine residue, a phenylalanine residue, a tyrosine residue, a tryptophan residue, an asparagine residue, a glutamine residue, a threonine residue, an aspartic acid residue, a glutamic acid residue, an arginine residue, a histidine residue, or a methionine residue,
X6 represents any amino acid residue,
X4 represents an isoleucine residue, a phenylalanine residue, a tyrosine residue, an asparagine residue, a histidine residue, or a methionine residue, and
Xk represents a group represented by -X6-X4-.

In a still more preferred aspect,
L represents a cyclized portion represented by 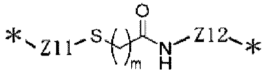 or 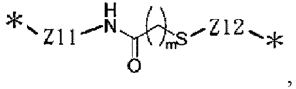 where Z11 and Z12 each independently represent a linking group, m represents an integer of 1 to 10, and * represents a position of an α-carbon atom of an amino acid main chain, and
L1 represents a cyclized portion represented by 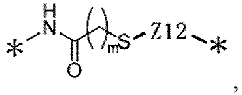 where Z12 represents a linking group, m represents an integer of 1 to 10, and * represents a position of an α-carbon atom of an amino acid main chain.

X1 preferably represents a glutamine residue.

X5 preferably represents a phenylalanine residue, a tyrosine residue, a tryptophan residue, a glutamine residue, a histidine residue, a thiol group-containing amino acid residue forming a cyclized portion, or a halocarboxyl group-containing amino acid residue forming a cyclized portion. X5 more preferably represents a phenylalanine residue, a tyrosine residue, a tryptophan residue, a histidine residue, a thiol group-containing amino acid residue forming a cyclized portion, or a halocarboxyl group-containing amino acid residue forming a cyclized portion. X5 particularly preferably represents a phenylalanine residue, a tyrosine residue, a tryptophan residue, or a histidine residue.

X6 preferably represents an alanine residue, a glycine residue, a leucine residue, an isoleucine residue, a valine residue, a phenylalanine residue, a tyrosine residue, a tryptophan residue, an asparagine residue, a glutamine residue, a serine residue, a threonine residue, an aspartic acid residue, a glutamic acid residue, an arginine residue, a histidine residue, a lysine residue, or a methionine residue. X6 more preferably represents an alanine residue, a glycine residue, a leucine residue, an isoleucine residue, a valine residue, a tyrosine residue, a tryptophan residue, an asparagine residue, a glutamine residue, a serine residue, a threonine residue, an aspartic acid residue, a glutamic acid residue, an arginine residue, a histidine residue, a lysine residue, or a methionine residue.

X4 preferably represents an alanine residue, a phenylalanine residue, or a tyrosine residue. X4 more preferably represents a phenylalanine residue or a tyrosine residue.

X2 preferably represents a leucine residue, an isoleucine residue, a phenylalanine residue, or a tyrosine residue. X2 more preferably represents a leucine residue, a phenylalanine residue, or a tyrosine residue. X2 particularly preferably represents a leucine residue or a phenylalanine residue.

Xn preferably represents a peptide residue consisting of two amino acid residues represented by Xn1-Xn2.

Xi preferably represents an amino acid residue represented by Xn1.

Xn1 preferably represents an alanine residue, a glycine residue, a leucine residue, an isoleucine residue, a valine residue, a phenylalanine residue, a tyrosine residue, a tryptophan residue, an asparagine residue, a glutamine residue, a serine residue, a threonine residue, an aspartic acid residue, a glutamic acid residue, an arginine residue, a histidine residue, a lysine residue, or a methionine residue. Xn1 more preferably represents an alanine residue, a leucine residue, an isoleucine residue, a valine residue, a phenylalanine residue, a tyrosine residue, a tryptophan residue, an asparagine residue, a glutamine residue, a serine residue, a threonine residue, an aspartic acid residue, a glutamic acid residue, an arginine residue, a histidine residue, a lysine residue, or a methionine residue.

Xn2 preferably represents an alanine residue, a glycine residue, a proline residue, a leucine residue, an isoleucine residue, a valine residue, a phenylalanine residue, a tyrosine residue, a tryptophan residue, an asparagine residue, a glutamine residue, a serine residue, a threonine residue, an aspartic acid residue, a glutamic acid residue, an arginine residue, a histidine residue, a lysine residue, a methionine residue, a thiol group-containing amino acid residue forming a cyclized portion, or a halocarboxyl group-containing amino acid residue forming a cyclized portion. Xn2 more preferably represents an alanine residue, a glycine residue, a leucine residue, an isoleucine residue, a valine residue, a phenylalanine residue, a tyrosine residue, a tryptophan residue, an asparagine residue, a glutamine residue, a serine residue, a threonine residue, an aspartic acid residue, a glutamic acid residue, an arginine residue, a histidine residue, a lysine residue, or a methionine residue. Xn2 particularly preferably represents an alanine residue, a leucine residue, a valine residue, a phenylalanine residue, a tyrosine residue, a tryptophan residue, an asparagine residue, a glutamine residue, a serine residue, a threonine residue, an aspartic acid residue, a glutamic acid residue, an arginine residue, a histidine residue, a lysine residue, or a methionine residue.

X3 preferably represents a phenylalanine residue, a tyrosine residue, or a tryptophan residue.

Y1 preferably represents a group represented by Ym-Y11-Yn,
Ym represents an amino acid residue or a peptide residue containing any 1 to 3 amino acid residues,
Y11 represents an alanine residue, a glycine residue, a tyrosine residue, an asparagine residue, a serine residue, a threonine residue, a glutamic acid residue, or a histidine residue, and
Yn represents a peptide residue consisting of any 5 amino acid residues.

Y11 preferably represents an asparagine residue.

Ym preferably represents an amino acid residue represented by Ym3, a peptide residue consisting of two amino acid residues represented by Ym2-Ym3, or a peptide residue consisting of three amino acid residues represented by Ym1-Ym2-Ym3. Ym more preferably represents a peptide residue consisting of two amino acid residues represented by Ym2-Ym3, or a peptide residue consisting of three amino acid residues represented by Ym1-Ym2-Ym3.

Ym1 preferably represents an alanine residue.

Ym2 preferably represents an alanine residue or a proline residue.

Ym3 preferably represents an alanine residue or a glutamine residue.

Yn preferably represents a peptide residue consisting of any five amino acid residues represented by Yn1-Yn2-Yn3-Yn4-Yn5.

Yn1 preferably represents an alanine residue or a phenylalanine residue. Yn1 more preferably represents a phenylalanine residue.

Yn2 preferably represents an alanine residue or an isoleucine residue. Yn2 more preferably represents an isoleucine residue.

Yn3 preferably represents an alanine residue or an asparagine residue. Yn3 more preferably represents an asparagine residue.

Yn4 preferably represents an alanine residue or a proline residue.

Yn5 preferably represents an alanine residue or a valine residue.

Y2 preferably represents a single bond.

Y3 preferably represents any 1 amino acid residue.

Y4 preferably represents any 1 amino acid residue.

Z1 and Z2 each independently represent a linking group, preferably represent an alkylene group having 1 to 10 carbon atoms, more preferably represent an alkylene group having 1 to 5 carbon atoms, and particularly preferably represent an alkylene group having 2 to 4 carbon atoms.

m preferably represents an integer of 1 to 5, more preferably represents an integer of 1 to 3, and particularly preferably represents an integer of 1.

Z11 and Z12 each independently represent a linking group, preferably an alkylene group having 1 to 10 carbon atoms, more preferably an alkylene group having 1 to 5 carbon atoms, and particularly preferably an alkylene group having 2 to 4 carbon atoms.

In the cyclic peptide, a plurality of amino acid residues contained in the polypeptide chain are bonded to each other by a covalent bond other than a main chain peptide bond to form a closed ring structure. In the cyclic peptide, the above-described partial structure consisting of a plurality of mutually bonded amino acid residues is referred to as a cyclized portion.

From the viewpoint of stability, the cyclized portion of the cyclic peptide needs to not contain a disulfide bond. Examples of the cyclized portion not containing a disulfide bond include a structure containing a thioether bond.

In a case of forming a thioether bond, for example, a side chain thiol group of an amino acid residue derived from an amino acid having a thiol group on a side chain can be reacted with a side chain chloroacetyl group of an amino acid residue derived from an amino acid having a chloroacetyl group on a side chain to form a thiol bond. Specifically, for example, a linear polypeptide can be cyclized by forming a thiol bond between a side chain thiol group of an L-homocysteine residue and a side chain chloroacetyl group of an N-ε-chloroacetyl-L-lysine residue.

It is noted that, instead of an amino acid having haloacetyl group such as chloroacetyl group on its side chain, it is also possible to use an amino acid having a halocarbonyl group with a greater number of methylene units than the haloacetyl group, such as a chloropropionyl group, on its side chain; however, an amino acid with a smaller number of methylene units is preferable, since a smaller number of methylene units results in higher cyclization efficiency. For example, an acetyl group (-C(=O)-CH₂-X; X is a halogen atom, and the number of methylene units is 1) is preferable since the cyclization efficiency is higher than that of a halopropionyl group (-C(=O)-(CH₂)₂-X; X is a halogen atom, and the number of methylene units is 2).

In the formation of the thioether, it is more preferable to use an amino acid having a halocarbonyl group in the side chain than to use an amino acid having a halocarbonyl group in the main chain, from the viewpoint of binding affinity to FGFR.

From the viewpoints of both stability and suppression of the generation of a racemized substance in the chemical synthesis step of peptides, homocysteine is preferable to cysteine as the amino acid on the thiol donor side. That is, the cyclized portion preferably includes a homocysteine residue.

From the viewpoint of binding affinity to an FGFR protein, the number of amino acid residues constituting the ring of the cyclic peptide is preferably 10 to 22, more preferably 14 to 22, and particularly preferably 15 to 20.

The amino acid used for the cyclic peptide may have a similar structure (generally regarded as similar) from the viewpoint of binding affinity to an FGFR protein. The similar structure includes an unnatural amino acid. The unnatural amino acid may be an unnatural amino acid in which a carbon chain is extended (such as homoserine), an unnatural amino acid to which a methyl group is added (such as N-methylalanine), or an unnatural amino acid which has been subjected to one or a plurality of modifications (such as phosphorylated tyrosine).

In the cyclic peptide, an amino acid at a site which is not particularly specified may be a natural amino acid, a non-natural amino acid, or an amino acid analogue (peptoid).

Specific examples of the cyclic peptide include a cyclic peptide having any one of amino acid sequences set forth in SEQ ID NOs: 37 to 450 and 452 to 454 described in Tables 4 to 6, or a cyclic peptide having an amino acid sequence in which 1 to 4 amino acids in any one of the amino acid sequences set forth in SEQ ID NOs: 37 to 450 and 452 to 454 described in Tables 4 to 6 have been substituted, deleted, or inserted, and having a binding ability to an FGFR protein.

Preferred specific examples of the cyclic peptide include a cyclic peptide having any one of amino acid sequences set forth in SEQ ID NOs: 37 to 51, 53 to 55, 57 to 61, 63 to 68, 70 to 89, 92, 93, 103, 104, 106 to 109, 114, 115, 117, 119, 121 to 123, 125 to 133, 136 to 181, 187 to 189, 194 to 199, 201 to 203, 205 to 207, 211 to 218, 220 to 235, 237 to 250, 254 to 270, 272, 275, 276, 278 to 291, 293 to 324, 328 to 337, 339, 341 to 354, 356, 358 to 360, 385, 386, 391, 392, 394 to 398, 400, 402, 403, 406, 407, 409 to 411, 430, 433 to 435, 437 to 444, 446 to 450, and 452 described in Tables 4 to 6, or a cyclic peptide having an amino acid sequence in which 1 to 4 amino acids in any one of the amino acid sequences set forth in SEQ ID NOs: 37 to 51, 53 to 55, 57 to 61, 63 to 68, 70 to 89, 92, 93, 103, 104, 106 to 109, 114, 115, 117, 119, 121 to 123, 125 to 133, 136 to 181, 187 to 189, 194 to 199, 201 to 203, 205 to 207, 211 to 218, 220 to 235, 237 to 250, 254 to 270, 272, 275, 276, 278 to 291, 293 to 324, 328 to 337, 339, 341 to 354, 356, 358 to 360, 385, 386, 391, 392, 394 to 398, 400, 402, 403, 406, 407, 409 to 411, 430, 433 to 435, 437 to 444, 446 to 450, and 452 described in Tables 4 to 6 have been substituted, deleted, or inserted, and having a binding ability to an FGFR protein.

More preferred specific examples of the cyclic peptide include a cyclic peptide having any one of amino acid sequences set forth in SEQ ID NOs: 37 to 42, 45, 47 to 51, 53 to 55, 60 to 61, 63, 65 to 68, 71 to 88, 92 to 93, 106, 108, 114 to 115, 117, 119, 121, 123, 125 to 133, 136 to 170, 172 to 180, 187 to 188, 194 to 199, 201, 203, 205 to 207, 211 to 212, 214 to 218, 220 to 235, 237 to 250, 254 to 260, 262 to 270, 272, 275, 278 to 290, 293, 295 to 324, 328 to 337, 339, 341 to 354, 356, 358 to 360, 391 to 392, 395, 397, 400, 402, 406, 409 to 411, 430, 433 to 435, 437 to 443, 446 to 450, and 452 described in Tables 4 to 6, or a cyclic peptide having an amino acid sequence in which 1 to 4 amino acids in any one of the amino acid sequences set forth in SEQ ID NOs: 37 to 42, 45, 47 to 51, 53 to 55, 60 to 61, 63, 65 to 68, 71 to 88, 92 to 93, 106, 108, 114 to 115, 117, 119, 121, 123, 125 to 133, 136 to 170, 172 to 180, 187 to 188, 194 to 199, 201, 203, 205 to 207, 211 to 212, 214 to 218, 220 to 235, 237 to 250, 254 to 260, 262 to 270, 272, 275, 278 to 290, 293, 295 to 324, 328 to 337, 339, 341 to 354, 356, 358 to 360, 391 to 392, 395, 397, 400, 402, 406, 409 to 411, 430, 433 to 435, 437 to 443, 446 to 450, and 452 described in Tables 4 to 6 have been substituted, deleted, or inserted, and having a binding ability to an FGFR protein.

The number of substituted, deleted, or inserted amino acids may be 1 to 4, but is preferably 1, 2, or 3, and more preferably 1 or 2.

The binding affinity to the FGFR protein is indicated by the amount of the cyclic peptide bound to the FGFR protein in a case where the cyclic peptide is allowed to act on a certain amount of the FGFR protein at a specific concentration. In the present disclosure, the measurement is performed using the ELISA method and the SPR method, but the measurement can be performed in the same manner by other methods (ITC, alphascreen, and the like). The FGFR protein may be a full-length protein, may be a partial domain, or may have an introduced mutation.

Regarding the molecular stability of the cyclic peptide, in the present disclosure, the measurement is conducted using reduction resistance and alkali resistance as indicators, but the same is also exhibited in the resistance to other stimuli (for example, X-ray resistance, γ-ray resistance, ultraviolet resistance, heat resistance, and chemical resistance). The molecular stability basically indicates that the molecule is more stable from the viewpoint of free energy.

The cyclic peptide may be modified with another substance as exemplified below.

The cyclic peptide may be imparted with 1 to 20 amino acids and/or a modified structure. From the viewpoint of the cost of chemical synthesis of peptides, it is preferable that the amino acid is not added. From the viewpoint of stability, it is preferable that the modified structure is imparted. Examples of the modified structure include acetylation at the N-terminus and structural analogs thereof, and amidation at the C-terminus and structural analogs thereof.

The cyclic peptide may be modified by, for example, phosphorylation, methylation, adenylylation, adenosine diphosphate (ADP) ribosylation, or glycosylation, according to the intended use.

**In** addition, from the viewpoints of water solubility, stability, ease of separation, and/or ease of tracking, a functional structure may be imparted to the cyclic peptide. Examples of the functional structure include water-soluble polymers such as polyethylene glycol (PEG), IgG and other proteins, various affinity tags (for example, a histidine (His) tag and a FLAG tag), an immobilized carrier (for example, Sepharose beads, magnetic beads, and various gels), a fluorescent dye, and a radioactive isotope.

An amino acid and/or a modified structure may be missing from the cyclic peptide. From the viewpoint of binding affinity to an FGFR protein, the number of amino acid residues to be deleted is required to be 4 or less, preferably 2 or less, and particularly preferably 0.

The cyclic peptide according to the embodiment of the present invention may be a salt. The salts are preferably salts with physiologically acceptable inorganic and organic acids and bases. Examples of appropriate acid salts include the following: acetate, adipate, benzoate, benzenesulfonate, butyrate, citrate, digluconate, dodecyl sulfate, formate, fumarate, glycolate, hemisulfate, heptanoate, hexanoate, hydrochloride, hydrobromide, hydroiodide, lactate, maleate, malonate, methane sulfonate, 2-naphthalenesulfonate, nicotinate, nitrate, pamoate, phosphate, picrate, pivalate, propionate, salicylate, succinate, sulfate, tartrate, tosylate, trifluoroacetate, and undecanoate. Salts derived from appropriate bases include an alkali metal (for example, sodium) salt, an alkaline earth metal (for example, magnesium) salt, an ammonium salt, and an N-(alkyl)₄⁺ salt.

### <Method for manufacturing cyclic peptide>

A method for manufacturing the cyclic peptide is not particularly limited, and the cyclic peptide can be manufactured by chemical synthesis, genetic engineering synthesis, or enzymatic synthesis by a cell-free translation system. From the viewpoint of purity, chemical synthesis is more preferable.

The peptide synthesis by chemical synthesis may be performed by a solid-phase synthesis method or a liquid-phase synthesis method. In the synthesis of a small amount, a solid phase synthesis method is preferable, and a solid phase synthesis method using an automatic peptide synthesizer is simple and preferable. In the large amount of synthesis, liquid phase synthesis is preferable from the viewpoint of workability. Examples of a guide for a large amount include 1 g or more, but the present invention is not limited thereto.

The solid phase synthesis of a peptide is known to those skilled in the art, and involves, for example, an esterification reaction between a hydroxyl group of a resin having a hydroxyl group and a carboxyl group of a first amino acid (usually a C-terminal amino acid of a desired peptide) in which an α-amino group is protected with a protective group. A known dehydration condensation agent such as 1-mesitylenesulfonyl-3-nitro-1,2,4-triazole (MSNT), dicyclohexylcarbodiimide (DCC), or diisopropylcarbodiimide (DIPCDI) can be used as an esterification catalyst. Next, the protective group of the α-amino group of the first amino acid is eliminated, a second amino acid in which all functional groups of a main chain except a carboxyl group are protected is added, and the carboxyl group is activated to bond the first amino acid and the second amino acid. Furthermore, the α-amino group of the second amino acid is deprotected, a third amino acid in which all functional groups of a main chain except a carboxyl group are protected is added, and the carboxyl group is activated to bond the second amino acid and the third amino acid. This process is repeated and in a case where a peptide having a desired length is synthesized, all functional groups are deprotected. Examples of the resin for solid phase synthesis include Merrifield resin, MBHA resin, Cl-Trt resin, SASRIN resin, Wang resin, Rink amide resin, HMFS resin, Amino-PEGA resin (Merck), HMPA-PEGA resin (Merck), and the like. These resins may be washed with a solvent (dimethylformamide (DMF), 2-propanol, methylene chloride, or the like) before use. Examples of the protective group for the α-amino group include a benzyloxycarbonyl (Cbz or Z) group, a tert-butoxycarbonyl (Boc) group, a fluorenylmethoxycarbonyl (Fmoc) group, a benzyl group, an allyl group, and an allyloxycarbonyl (Alloc) group. The Cbz group can be deprotected by hydrofluoric acid, hydrogenation, or the like, the Boc group can be deprotected by trifluoroacetic acid (TFA), and the Fmoc group can be deprotected by a treatment with piperidine. The protection of an α-carboxyl group can be carried out using a methyl ester, an ethyl ester, a benzyl ester, a tert-butyl ester, a cyclohexyl ester, or the like. As for other functional groups of amino acids, a hydroxyl group of serine or threonine can be protected with a benzyl group or a tert-butyl group, and a hydroxyl group of tyrosine can be protected with a 2-bromobenzyloxycarbonyl group or a tert-butyl group. An amino group in a side chain of lysine and a carboxyl group of glutamic acid or aspartic acid can be protected in the same manner as the α-amino group and the α-carboxyl group. The activation of the carboxyl group can be performed using a condensing agent. Examples of the condensing agent include dicyclohexylcarbodiimide (DCC), diisopropylcarbodiimide (DIPCDI), 1-ethyl-3-(3-dimethylaminopropyl) carbodiimide (EDC or WSC), (1H-benzotriazol-1-yloxy)tris(dimethylamino)phosphonium hexafluorophosphate (BOP), and 1-[bis(dimethylamino)methyl]-1H-benzotriazolium-3-oxide hexafluorophosphate (HBTU). Cleavage of a peptide chain from the resin can be carried out by a treatment with an acid such as TFA or hydrogen fluoride (HF).

Genetic engineering synthesis is a method of introducing a gene into cells to synthesize a peptide. As the cells, bacteria, yeasts, nematode cells, insect cells, animal cells (such as mammalian cells), or the like are used.

For example, the peptide can be synthesized by introducing an unnatural amino acid using a four-base codon method. In addition, the cyclic peptide can be synthesized by synthesizing a chain-like peptide and cyclizing the chain-like peptide by reacting a crosslinking functional group of a side chain of an amino acid residue introduced into the cyclic portion.

The cell-free translation system is also called a cell-free protein synthesis system, and is a translation system that uses components present in cells such as Escherichia coli without using cells such as Escherichia coli as they are, and there are systems using a cell extract and systems (reconstituted cell-free translation system) using a reaction solution obtained by purifying and reconstituting each component of a cell extract.

In systems using a cell extract, examples of the cell extract include an Escherichia coli extract, a wheat germ extract, a rabbit erythrocyte extract, and an insect cell extract.

The reconstituted cell-free translation system can be constructed with ribosomal proteins, aminoacyl tRNA synthetase (ARS), ribosomal RNA, amino acids, GTP, ATP, translation initiation factor (IF), elongation factor (EF), termination factor (RF), ribosome recycling factor, and other factors required for translation, which has been purified.

In the translation system, energy may be continuously supplied using dialysis. RNA polymerase may be added to perform transcription from DNA.

Examples of commercially available cell-free translation systems include systems derived from Escherichia coli, such as RTS-100 (registered trademark) manufactured by Roche Diagnostics K.K., systems derived from wheat germ extract, such as products manufactured by Zyagen Corporation and CellFree Sciences Co., Ltd., and reconstituted translation systems, such as PURESYSTEM (registered trademark) manufactured by Post Genome Institute, Purefrex (registered trademark) manufactured by GeneFrontier Corporation, and PURExpress (registered trademark) In Vitro Protein Synthesis Kit manufactured by New England Biolabs, Inc.

### <Cyclic peptide complex>

According to the present invention, there is provided a cyclic peptide complex or a salt thereof, in which two or more molecules of the cyclic peptide or a salt thereof according to the embodiment of the present invention are linked by a linker. The number of molecules of the cyclic peptide linked by a linker or a salt thereof is not particularly limited as long as it is 2 or more, but is, for example, 2 to 10, preferably 2 to 5, more preferably 2 to 4, still more preferably 2 or 3, and particularly preferably 2.

FGFR is a single-pass transmembrane receptor tyrosine kinase, and upon binding of FGF, FGFR dimerizes, bringing the intracellular kinase domains of FGFR into proximity, leading to autophosphorylation and promoting cell proliferation induction and/or differentiation state regulation. Therefore, it is considered that the multimerization of the cyclic peptide having binding affinity to an FGFR protein (hereinafter, the multimerized cyclic peptide will be referred to as a cyclic peptide complex) through a linker structure and the action of the cyclic peptide complex on a cell expressing FGFR will cause the multimerization of FGFR through the cyclic peptide complex, whereby FGFR can be phosphorylated. In this case, in consideration of the fact that the phosphorylation is mediated by the proximity between the intracellular kinase domains of FGFR, it is considered that it is preferable to multimerize FGFR at an appropriate distance and alignment.

In addition, the FGFR phosphorylation intended to maintain the proliferation or differentiation state of cells expressing FGFR or the like may require a plurality of cyclic peptides. The plurality of cyclic peptides contained in the cyclic peptide complex may be complexed by a covalent bond or may be bonded by a non-covalent bond (for example, complex formation, affinity bond, or nucleic acid hybridization). The above-described bond may be a non-covalent bond from the viewpoint of synthetic cost, but a covalent bond (for example, an amide bond or various click chemistries) is preferable from the viewpoint of complex stability.

The plurality of cyclic peptides may be complexed by a direct bond between the cyclic peptides, or may be complexed through another molecule. The plurality of cyclic peptides contained in the cyclic peptide complex may have the same structure or may have different structures. From the viewpoints of cell proliferation-promoting effect and/or cell differentiation state regulation, the cyclic peptide complex may have different structures, but from the viewpoint of the synthesis cost, it is preferable to have the same structure.

The length of the linker is not particularly limited, but is preferably 10 to 200 Å and more preferably 20 to 100 Å, from the viewpoint of FGFR phosphorylation.

In calculating the length of the linker, a unit distance of 1.33 Å (4 Å per one amino acid residue) has been calculated in consideration of the bending of the molecular chain. By using this unit distance, the length of the linker can be calculated based on the molecular structure.

In the cyclic peptide complex, from the viewpoint of FGFR phosphorylation, the linker can be used regardless of whether the linker is bound to the N-terminal or the C-terminal of the cyclic peptide, but it is more preferable that the linker is bonded to the C-terminal thereof.

In the cyclic peptide complex, from the viewpoint of FGFR phosphorylation, the linker structure may be polyethylene glycol (PEG), an alkyl chain (polyethylene), a polypeptide, a polyester, polyacrylamide, polycarbonate, polypropylene, polystyrene, and/or polyurethane. In addition, the linker structure may form a salt. In a case where the linker structure forms a salt, the salt is preferably the above-described salt. From the viewpoint of water solubility of the cyclic peptide complex, polyethylene glycol (PEG), an alkyl chain (polyethylene), a polypeptide, polyester, and/or polyacrylamide is more preferable.

The cyclic peptide complex can be manufactured, for example, by linking two cyclic peptides each having one amino group using a linker molecule having two NHS-activated carboxyl groups (hereinafter, referred to as a Bis-NHS linker). Bis-NHS linkers having various linker lengths are sold by various companies, and examples thereof include BS(PEG)5 (PEGylated bis(sulfosuccinimidyl)suberate) (Thermo, 21581) and Bis(NHS)PEG9 (Tokyo Chemical Industry Co., Ltd., B4688). The linking of the cyclic peptide using a Bis-NHS linker can be performed by mixing the cyclic peptide and the linker in water at a pH of about 7.

In addition, by using a branched amino acid having a plurality of amino groups in solid phase peptide synthesis and liquid phase synthesis, it is possible to manufacture a cyclic peptide complex. Examples of the branched amino acid include lysine, 2,4-diaminobutanoic acid, and the like. In this case, the branched amino acid is linked to a solid phase resin or a tag for liquid phase synthesis, then a plurality of amino groups are deprotected, and the peptide chain is extended with respect to both the solid phase resin and the tag for liquid phase synthesis, whereby it is possible to synthesize a cyclic peptide complex on the solid phase resin or on the tag for liquid phase synthesis. The peptide chains may be extended simultaneously on all amino groups or may be extended one by one.

The cyclic peptide complex according to the embodiment of the present invention may be modified with another substance. The other substances are the same as those described above regarding the modification of the cyclic peptide.

### <Use of cyclic peptide and cyclic peptide complex>

The cyclic peptide or a salt thereof according to the embodiment of the present invention and the cyclic peptide complex or a salt thereof according to the embodiment of the present invention can be used for functional analysis, functional regulation, labeling, proliferation promotion, differentiation state regulation, purification, or the like of a biological substance (such as a protein, a cell, or a tissue) including an FGFR protein. Among these, the proliferation-promoting effect of the cyclic peptide complex or a salt thereof according to the embodiment of the present invention contributes to the reduction of the production cost of various cell therapy products and cultured meat, and thus has high industrial value. In particular, in cultured meat, it is possible to perform commercial production by substituting a growth factor, which accounts for most of the production cost (up to 96% or more), with the cyclic peptide complex or a salt thereof according to the embodiment of the present invention.

From the viewpoints of binding affinity, functional regulation, labeling efficiency, purification, proliferation promotion, and/or differentiation state regulation, the target on which the cyclic peptide or a salt thereof, or the cyclic peptide complex or a salt thereof is to be acted may be an FGFR protein, and may be another protein, a compound, and/or a cell. However, an FGFR protein and/or a cell that expresses an FGFR protein, a cell secretion therefrom and/or a cell lysate thereof, or a cell in which a proliferation effect or an undifferentiated state maintenance effect by bFGF is known is preferable. FGFR2 or FGFR4 protein and/or a cell that expresses FGFR2 or FGFR4, a cell secretion therefrom, and/or a cell lysate thereof is more preferable. FGFR2 protein and/or a cell that expresses FGFR2, a cell secretion therefrom, and/or a cell lysate thereof is particularly preferable.

The target on which the cyclic peptide or a salt thereof, or the cyclic peptide complex or a salt thereof is to be acted may be a substance derived from a human or a substance derived from a mouse, a cow, or another animal. From the viewpoint of binding affinity, functional regulation, labeling efficiency, purification, proliferation promotion, and/or differentiation state regulation, a substance derived from an animal having a high homology of an amino acid sequence constituting FGFR to humans is preferable. Examples of animals having a high homology of amino acids constituting FGFR to humans include cattle, pigs, birds, and bluefin tuna.

The cyclic peptide or a salt thereof, or the cyclic peptide complex or a salt thereof may be used by being dissolved in an aqueous solution or an organic solvent, or may be used by being bonded to an immobilized carrier (for example, a plate or beads). In a case of being used by being bonded to an immobilized carrier, it is preferable to introduce a reactive group for bonding (for example, an amino group, a thiol group, or biotin) into the cyclic peptide and/or the cyclic peptide complex or a salt thereof from the viewpoints of bonding efficiency and/or expression of target performance.

Specifically, the cyclic peptide or a salt thereof according to the embodiment of the present invention and the cyclic peptide complex or a salt thereof according to the embodiment of the present invention can be used as a culture medium composition, a culture medium additive, a material for purification, a material for labeling, a material for cell regulation, or a material for accumulation.

**In** a case where a cyclic peptide or a salt thereof, or a cyclic peptide complex or a salt thereof is used as the culture medium composition, the culture medium composition can be prepared by formulating the constituent components according to a conventional method, and the form of the culture medium composition is not particularly limited as long as a desired effect such as a cell proliferation-promoting effect and/or cell regulation is obtained, and it can be prepared, for example, in the form of a liquid culture medium, a semi-fluid culture medium, or a solid culture medium. In addition, the culture medium composition according to the embodiment of the present invention may be prepared in a powdery form. The transportation and the storage can be extremely easy by preparing in the powdery form. In addition, by adding sterile water and/or agar or the like at the time of use, it is possible to easily prepare a liquid, semi-liquid, or solid culture medium. In a case of preparing a liquid, semi-liquid, or solid culture medium, from the viewpoint of FGFR phosphorylation, the preparation concentration of the cyclic peptide or a salt thereof, or a cyclic peptide complex or a salt thereof is preferably 0.001 to 100 nmol/L, more preferably 0.01 to 10 nmol/L, and particularly preferably 0.1 to 10 nmol/L.

In addition, the culture medium composition according to the embodiment of the present invention can be used for any culture method such as adhesion culture, suspension culture, embedding culture, and tissue culture.

In a case where the cyclic peptide or a salt thereof, or the cyclic peptide complex or a salt thereof is used as the culture medium composition, in addition to the cyclic peptide or a salt thereof, or the cyclic peptide complex or a salt thereof according to the embodiment of the present invention, components generally used for cell culture, for example, amino acids, vitamins, buffering materials, inorganic salts, carbon sources, serum, serum substitutes, and the like can be appropriately adopted.

In a case where the cyclic peptide or a salt thereof, or the cyclic peptide complex or a salt thereof is used as the culture medium composition, is can be used by being dissolved in a culture medium. In a case of being used by being dissolved in a culture medium, from the viewpoint of FGFR phosphorylation, the dissolution concentration is preferably 0.001 to 100 nmol/L, more preferably 0.01 to 10 nmol/L, and particularly preferably 0.1 to 10 nmol/L.

In a case where the cyclic peptide or a salt thereof, or the cyclic peptide complex or a salt thereof is used by being dissolved in a culture medium, the culture medium may be any culture medium as long as it is generally used for cell culture, but from the viewpoints of cell proliferation-promoting effect and/or cell regulation, a culture medium in which components are prepared to be suitable for culturing cells to be used is more preferable.

The culture medium may contain serum, but in a case of being used for manufacturing a cell product such as a cell therapy product or cultured meat, it is preferably a serum-free medium from the viewpoint of safety. The serum-free medium does not contain serum, but may contain purified components derived from serum or may contain a recombinant protein of a serum-derived component. The serum-free medium may contain a serum substitute, and examples thereof include a medium appropriately containing serum albumin, transferrin, fatty acids, collagen precursors, trace elements, 2-mercaptoethanol, 3-thiol glycerol, or an equivalent thereof. Such serum substitute can be prepared, for example, by the method described in WO 98/30679A. The serum substitute also includes the cyclic peptide or a salt thereof, or the cyclic peptide complex or a salt thereof according to the embodiment of the present invention. A commercially available product may be used as the serum substitute. Examples of such commercially available serum substitute include Knockout^{™} Serum Replacement (Life Technologies Corporation), Commercially-defined Lipid concentrated (Life Technologies Corporation), Glutamax^{™} (Life Technologies Corporation), B27 (Life Technologies Corporation), and N2 (Life Technologies Corporation), but the serum substitute is not limited thereto.

In a case where the cyclic peptide or a salt thereof, or the cyclic peptide complex or a salt thereof is used as a culture medium additive, the cyclic peptide or a salt thereof, or the cyclic peptide complex or a salt thereof can be used in a case of adding the cyclic peptide or a salt thereof, or the cyclic peptide complex or a salt thereof to the culture medium. The culture medium additive according to the embodiment of the present invention may contain any additive, for example, a stabilizer, an isotonic agent, a pH adjuster, or the like in an appropriate amount, in addition to the cyclic peptide or a salt thereof, or a cyclic peptide complex or a salt thereof according to the embodiment of the present invention, as long as the desired effect such as a cell proliferation-promoting effect and/or cell regulation is not impaired.

The culture medium additive according to the embodiment of the present invention may be in any form as long as a desired effect can be obtained, and examples thereof include a solution, a solid form, a powdery form, and the like. From the viewpoint of reducing transportation cost, the solid form or the powdery form is preferable. In a case where the solid form or the powdery form is used, the additive may be dissolved using an appropriate buffer solution, solvent, or the like to have a desired concentration, or the solid form or the powdery form of the additive may be used as it is. In a case where the culture medium additive is a solution, it is preferable that the solution is subjected to a sterilization treatment such as filtration sterilization using a membrane filter or the like.

The number of times and the timing of adding the culture medium additive according to the embodiment of the present invention to the culture medium are not particularly limited, and the addition may be performed before cell culture, during cell culture, or after cell culture, but from the viewpoint of proliferation promotion and/or differentiation state regulation, the addition is preferably performed before cell culture and/or during cell culture.

In a case where the cyclic peptide or a salt thereof, or the cyclic peptide complex or a salt thereof is used as a culture medium composition or a culture medium additive, the culture medium composition or the culture medium additive can be used for producing a cell therapy product or cultured meat. The method for manufacturing a cell therapy product or cultured meat can include, for example, a step of culturing cells using a culture medium containing a cyclic peptide or a salt thereof, or a cyclic peptide complex or a salt thereof.

The cultured meat refers to meat manufactured through cell culture. It is an innovative technology that produces animal meat from a culture tissue using cell engineering, instead of a conventional method of raising livestock. The production process includes culturing animal cells that are produced to grow in muscle tissue or fat. With this technique, a food rich in a protein that is materially and nutritionally equivalent to a food based on meat or fish in the related art can be obtained. In addition to the clear animal welfare concerns, the breeding of livestock animals requires a large amount of resources because the use of water and land for the cultivation of feed crops continues to increase. In addition, due to the increased awareness of the emission of greenhouse gases, the destruction of forests, the ecological impact of antibiotic resistance and pollution, and the production of meat, and the increased interest in animal welfare, there is a proposal for "cultured meat", "clean meat", or "cultured meat" to replace the consumption of meat in the related art with "laboratory-cultured meat".

In general, the meat refers to a collection of muscle fibers, connective tissues, and fat. It is preferable that the cultured meat mimics the structure of the meat, but the cultured meat may not contain all components of so-called meat, and is only required to contain cultured cells selected from the group consisting of fibroblasts, adipose tissue-derived cells, and muscle tissue-derived cells. It is more preferable to contain a culture product of a plurality of types of cells.

The cultured meat may contain an extracellular matrix in addition to the cultured cells selected from the group consisting of fibroblasts, adipose tissue-derived cells, and muscle tissue-derived cells. The method for manufacturing cultured meat includes, for example, a step of culturing cultured cells selected from the group consisting of the following fibroblasts, adipose tissue-derived cells, and muscle tissue-derived cells, and a step of recovering and accumulating the cultured cells. The method for manufacturing cultured meat may further include a differentiation induction step or a culture step after accumulation.

In a case where the cyclic peptide or a salt thereof, or the cyclic peptide complex or a salt thereof is used as a culture medium composition or a culture medium additive, the culture of cells can be performed by seeding cells in a culture medium containing the cyclic peptide or a salt thereof, or the cyclic peptide complex or a salt thereof. The culture is performed under conditions well known in the technical field, for example, in a 37°C CO₂ incubator. The culture may be an adherent culture or a suspension culture. The proliferated cells can be recovered as a culture product of cells by a trypsin treatment or the like, and further subculture may be performed after the recovery. In the culture of cells, cells can also be seeded and cultured on a peelable construction. The construction to which the proliferated cells are attached can be recovered as a culture product. Such construction can be constructed with an extracellular matrix such as collagen, elastin, fibronectin, laminin, or entactin, and a cultured meat may be molded by accumulating the construction to which cells are attached.

The accumulation step includes molding the culture product of one or a plurality of types of the recovered cells. The culture product molded in the accumulation step may be a single piece of meat such as steak, may be a carcass, or may be minced. The accumulation step includes accumulating the culture product of cells together with at least one substance selected from the group consisting of other cells, blood, and tissues. The other cells may be cultured cells or cells collected from an animal. More specifically, the cells can be molded together with other cells cultured in a culture medium containing the cyclic peptide or a salt thereof, or the cyclic peptide complex or a salt thereof according to the embodiment of the present invention. As an example, the muscle tissue-derived cells cultured in a culture medium containing the cyclic peptide or a salt thereof, or the cyclic peptide complex or a salt thereof according to the embodiment of the present invention can be accumulated with the adipose tissue-derived cells and/or fibroblasts cultured in a culture medium containing the cyclic peptide or a salt thereof, or the cyclic peptide complex or a salt thereof according to the embodiment of the present invention. Furthermore, co-culture can be performed after the accumulation. For example, a culture product of one or more types of recovered cells can be mixed and seeded on an extracellular matrix for co-culture. Collagen, elastin, fibronectin, laminin, entactin, and the like can be used as the extracellular matrix. In this case, a culture medium containing the cyclic peptide or a salt thereof, or the cyclic peptide complex or a salt thereof according to the embodiment of the present invention can be also used as the culture medium.

In the accumulation step, the culture product of one or more types of the recovered cells may be accumulated with blood and/or tissue. The tissue may be acquired from an animal or may be cultured. As an example, the cultured meat may be manufactured by accumulating blood, adipose tissue, muscle tissue, or the like separated in the processing process of the meat, with the culture product.

The differentiation induction step may be performed after cell culture, or may be performed before, during, or after the accumulation step. By the differentiation induction step, mononuclear muscle satellite cells and myoblasts can be differentiated into multinuclear myotube cells, and further matured as muscle fibers. The differentiation induction may be performed by a method known in the present technical field, and as an example, a method of culturing under a high carbon dioxide concentration is known, and as an example, the differentiation into myotube cells can be promoted by culturing in a CO₂ atmosphere of 5% to 10% (v/v).

The culture medium containing a cyclic peptide or a salt thereof, or a cyclic peptide complex or a salt thereof can be used for culturing any animal cell. From the viewpoint of manufacturing cultured meat, cells derived from livestock such as cattle, pig, goat, sheep, rabbit, chicken, ostrich, and duck can be used. In particular, in a case where bovine cells are placed, the cells may be of any breed among Holstein, Jersey, Japanese Black, Japanese Brown, Japanese Shorthorn, Japanese Polled, or crossbreeds thereof, but cells of beef breed such as Japanese Black, Japanese Brown, Japanese Shorthorn, and Japanese Polled are preferable from the viewpoint of manufacturing the meat.

The culture medium containing the cyclic peptide or a salt thereof, or the cyclic peptide complex or a salt thereof can also be used for culturing a tissue in which cells are aggregated. The animal cells may be primary cells acquired from an animal or subculture cells subcultured from the primary cells, or may be cells that has been subjected to immortalization or gene-modified cells in which a gene has been deleted or inserted. The primary cells can be obtained by culturing an animal tissue in a culture medium. The primary cells may be cells differentiated from stem cells such as stem cells. From the viewpoint of manufacturing a cell therapy product, it is preferable to culture somatic stem cells or induced pluripotent stem cells, which include mesenchymal stem cells. From the viewpoint of manufacturing cultured meat, it is preferable to culture cells selected from the group consisting of fibroblasts, adipose tissue-derived cells, and muscle tissue-derived cells.

Fibroblasts are cells that constitute connective tissues and produce extracellular matrices such as collagen and elastin. Fibroblasts present in muscle are particularly referred to as muscle fibroblasts. The muscle fibroblast forms connective tissue surrounding a bundle of muscle fibers in skeletal muscle. The muscle fibroblast expresses α-SMA, produces an extracellular matrix, and can accumulate fat, thereby contributing to the crispness and the taste.

The adipose tissue-derived cells are cells constituting the adipose tissue, and are cells that are separated from the adipose tissue and cultured. The adipose tissue-derived cell is at least one cell selected from the group consisting of an adipose stem cell, a multilocular adipocyte, and a unilocular adipocyte. The adipose stem cell is a mesenchymal stem cell having a differentiation potency into various cells, and can be differentiated into a muscle cell, an adipocyte, and a connective tissue cell. The multilocular adipocyte is also known as a brown adipocyte and contributes to fat burning in a living body. The unilocular adipocyte is also known as a white adipocyte, and can store fat droplets in the cell. The adipose tissue-derived cell contains fat, and thus contributes to the taste of the meat.

The muscle tissue-derived cells are cells constituting the muscle tissue, and are cells that are separated from the muscle tissue and cultured. Examples of the muscle tissue-derived cells include myoblasts, muscle satellite cells, and myotube cells. However, since myotube cells do not have proliferative properties, from the viewpoint of proliferation, myoblasts and/or muscle satellite cells are preferable. The muscle satellite cells are somatic stem cells contained in muscle, and can proliferate and differentiate into myoblasts. The myoblasts are cells from which muscle fibers are derived, and are mononuclear cells having proliferative properties. In a case where the myoblasts are differentiated, the myoblasts fuse with each other to form multinucleated myotube cells, and further mature to form muscle fibers. The muscle fibers are composed of myofibrils as structural units, which consist of actin fibers and myosin fibers, which are proteins constituting muscles. Depending on the isoform of myosin, muscle fibers are classified into red muscle fibers (type I and type IIA) and white muscle fibers (type IIB), and they contribute to differences in the taste of meat.

In a case where the cyclic peptide or a salt thereof, or the cyclic peptide complex or a salt thereof is used as a material for purification, the cyclic peptide may be used by being bound to a solid phase carrier or may be used without being bound. As a method of using without being bound, there is a method of imparting a structure for separation (for example, a biotin tag, an antibody epitope, or a polar residue for electrostatic separation) to a cyclic peptide, or a method of imparting a toxin to a cyclic peptide to kill cells other than the target cells.

In a case where a cyclic peptide or a salt thereof, or a cyclic peptide complex or a salt thereof is used as a material for labeling, a fluorescent dye, a radioactive isotope, a nuclear polarized molecule, an affinity tag (for example, biotin or FLAG tag), or enzymes (for example, luciferase) may be used as a labeling group.

In a case where the cyclic peptide or a salt thereof, or the cyclic peptide complex or a salt thereof is used as a material for cell regulation, the cyclic peptide may be used by being bound to a solid phase carrier (for example, a flask or a plate), or may be used without being bound. Examples of the cell regulation include promotion of FGFR phosphorylation, inhibition of FGFR phosphorylation, promotion of cell proliferation, inhibition of cell proliferation, promotion of cell differentiation, and suppression of cell differentiation. In addition, by linking the cyclic peptide to another cell stimulator, it is possible to exhibit the effect of the cell stimulator linked selectively to a cell having FGFR.

In a case where the cyclic peptide or a salt thereof, or the cyclic peptide complex or a salt thereof is used as a material for accumulating a specific molecule in the vicinity of FGFR, it is necessary to link the molecule to be accumulated to the cyclic peptide. The linking may be 1 molecule-to-1 molecule or 1 molecule-to-multiple molecules, and the linking may be a reversible bond or an irreversible bond.

### Examples

The cyclic peptide and the like according to the present disclosure will be described in more detail below with reference to specific examples. The materials, treatment procedures, and the like shown in the specific examples below can be changed as appropriate without departing from the gist of the present disclosure. The scope of the cyclic peptide and the like according to the present disclosure should not be construed as being limited by the following specific examples. In the following description, unless otherwise specified, the synthesis, treatment, manufacture, and the like were performed at room temperature (25°C ± 3°C). The percentage related to the substance concentration is based on mass.

### <Example 1: Acquisition of FGFR-binding candidate peptide using mRNA display method>

Candidate sequences of a cyclic peptide that binds to FGFR were acquired using an mRNA display method.

The sequence set forth in SEQ ID NO: 1 was used as a library of the mRNA display method. In this library, translation starts from the start codon (ATG) at the positions 86 to 88 from the 5' terminal, a base group (NNNTGT(NNN)_{6 to 18}TAGNNN (NNN is a trimer oligonucleotide, where N's each independently represent A, T, G, or C)) from the position 107 is a random sequence, and a base group located on the 3' terminal side of the random sequence is a binding site of a puromycin linker and a stop codon. A base group before the position 85 from the 5' terminal is a sequence necessary for transcription and translation initiation, such as a T7 promoter sequence or a Shine-Dalgarno sequence. Since the triplet TAG in the present random sequence is assigned to the codon of chloroacetylated lysine, the peptide encoded by the present random sequence is a cyclic peptide in which a thiol group of cysteine and a chloroacetyl group of chloroacetylated lysine spontaneously form a thioether bond. In the nucleic acids having the sequences set forth in SEQ ID NO: 1, the trimer oligonucleotide represented by NNN is an equal amount mixture of trimer nucleotides corresponding to 18 types of codons shown in Table 1, in which one type of codon is assigned to one type of amino acid. Regarding the (NNN)_{6 to 18} part, 13 types of libraries having different repetition numbers ranging from 6 to 18 were individually prepared, and mRNA display was performed for each of the libraries.

**[Table 3]**

| Amino acid (one-letter abbreviation) | Assigned codon | Amino acid (one-letter abbreviation) | Assigned codon |
|---|---|---|---|
| A | GCT | N | AAC |
| I | ATC | Q | CAG |
| L | CTG | R | CGT |
| V | GTT | H | CAT |
| F | TTC | K | AAA |
| W | TGG | D | GAC |
| Y | TAC | E | GAA |
| S | TCT | G | GGT |
| T | ACT | P | CCG |

SEQ ID NO: 1:

GAAATTAATACGACTCACTATAGGGAGACCACAACGGTTTCCCTCTAGAAATAAT
TTTGTTTAACTTTAAGAAGGAGATATACATATGGTTAAGAAAACAAAAACANNNT
GT(NNN)6 to
18TAGNNNGGTGGCTCTGGCGGTAGCAGGACGGGGGGCGGCGGGGGGTAAATAAA
TAAGCTTGAGTAT

(NNN is a trimer oligonucleotide, where N's each independently represent A, T, G, or C)

The library set forth in SEQ ID NO: 1 was produced by performing overlap extension PCR. Specifically, a library was produced by mixing three types of DNAs, the DNA set forth in SEQ ID NO: 2, the DNA set forth in SEQ ID NO: 3, and the DNA set forth in any of SEQ ID NOs: 4 to 16, such that the concentrations thereof were 3 µmol/L, 1 µmol/L, and 1 µmol/L, repeatedly performing, after 98°C/30 seconds, three steps of 98°C/10 second, 60°C/10 seconds, and 72°C/10 seconds for 7 cycles in the presence of Platinum^{™} SuperFi II DNA Polymerase (Thermo, 12361010), and finally performing the treatment of 72°C/5 minutes, thereby linking the three DNAs. The produced library was purified and diluted to 10 ng/µL. In the DNA set forth in SEQ ID NOs: 4 to 17, the trimer oligonucleotide represented by NNN is an equal amount mixture of trimer nucleotides corresponding to 18 types of codons shown in Table 1, in which one type of codon is assigned to one type of amino acid.
SEQ ID NO: 2: 
GAAATTAATACGACTCACTATAGGGAGACCACAACGGTTTCCCTCTAGAAATAAT
TTTGTTTAACTTTAAGAAGGAGATATACATATGGTTAAAAAAACAAAAAC
SEQ ID NO: 3: 
ATACTCAAGCTTATTTATTTATTACCCCCCGCCGCCCCCCGTCCTGCTACCGCCAG
AACCACC
SEQ ID NO: 4: 
AAGAAGGAGATATACATATGGTTAAAAAAACAAAAACANNNTGT(NNN)6TAGNN
NGGCGGTTCTGGCGGTAGC
SEQ ID NO: 5: 
AAGAAGGAGATATACATATGGTTAAAAAAACAAAAACANNNTGT(NNN)7TAGNN
NGGCGGTTCTGGCGGTAGC
SEQ ID NO: 6: 
AAGAAGGAGATATACATATGGTTAAAAAAACAAAAACANNNTGT(NNN)8TAGNN
NGGCGGTTCTGGCGGTAGC
SEQ ID NO: 7: 
AAGAAGGAGATATACATATGGTTAAAAAAACAAAAACANNNTGT(NNN)9TAGNN
NGGCGGTTCTGGCGGTAGC
SEQ ID NO: 8: 
AAGAAGGAGATATACATATGGTTAAAAAAACAAAAACANNNTGT(NNN)10TAGNN
NGGCGGTTCTGGCGGTAGC
SEQ ID NO: 9: 
AAGAAGGAGATATACATATGGTTAAAAAAACAAAAACANNNTGT(NNN)11TAGNN
NGGCGGTTCTGGCGGTAGC
SEQ ID NO: 10: 
AAGAAGGAGATATACATATGGTTAAAAAAACAAAAACANNNTGT(NNN)12TAGNN
NGGCGGTTCTGGCGGTAGC
SEQ ID NO: 11: 
AAGAAGGAGATATACATATGGTTAAAAAAACAAAAACANNNTGT(NNN)13TAGNN
NGGCGGTTCTGGCGGTAGC
SEQ ID NO: 12: 
AAGAAGGAGATATACATATGGTTAAAAAAACAAAAACANNNTGT(NNN)14TAGNN
NGGCGGTTCTGGCGGTAGC
SEQ ID NO: 13: 
AAGAAGGAGATATACATATGGTTAAAAAAACAAAAACANNNTGT(NNN)15TAGNN
NGGCGGTTCTGGCGGTAGC
SEQ ID NO: 14: 
AAGAAGGAGATATACATATGGTTAAAAAAACAAAAACANNNTGT(NNN)16TAGNN
NGGCGGTTCTGGCGGTAGC
SEQ ID NO: 15: 
AAGAAGGAGATATACATATGGTTAAAAAAACAAAAACANNNTGT(NNN)17TAGNN
NGGCGGTTCTGGCGGTAGC
SEQ ID NO: 16:

AAGAAGGAGATATACATATGGTTAAAAAAACAAAAACANNNTGT(NNN)18TAGNN
NGGCGGTTCTGGCGGTAGC

Using Recombinant Human FGFR2 alpha (IIIc) Fc Chimera Protein, CF (R&D systems, 712-FR-050) as FGFR, immobilization (FGFR concentration during immobilization: 1 µg/µL) was performed on magnetic beads (NHS Mag Sepharose, Cytiva, 28951380) according to a protocol designated by the manufacturer (Cytiva). In addition, to remove the cyclic peptide bound to the magnetic beads, IgG1 Fc, Human, recombinant (FUJIFILM Wako Pure Chemical Corporation, 098-07141) was immobilized on the magnetic beads in the same manner as described above.

After a step of bringing the library into contact with the magnetic beads-immobilized FGFR and incubating the mixture was repeated a total of 8 rounds, the sequence of the peptide bound to FGFR was identified using a sequencer.

The specific procedure of each round is as follows.

First, the produced library (SEQ ID NO: 1) was reacted at 37°C for 30 minutes in the presence of T7 RNA Polymerase (TaKaRa, 2540A) to produce library transcriptions. This DNA fragment was purified and diluted to 10 µmol/L.

Next, the library transcript (final concentration: 5 µmol/L) and the puromycin linker set forth in SEQ ID NO: 17 (final concentration: 10 µmol/L) were mixed in a TBS buffer (1.25 mmol/L Tris, 25 mmol/L NaCl, pH: 7.5), then heated at 95°C for 5 minutes, and irradiated with UV (365 nm) on ice to produce a complex of the library transcript and the puromycin linker.

SEQ ID NO: 17: (Psoralen C6)-UACCCCCCGCCGCCCCCCGUCCU-(Sp18)-(Sp18)-(Sp18)-(Sp18)-CC-(Puro)

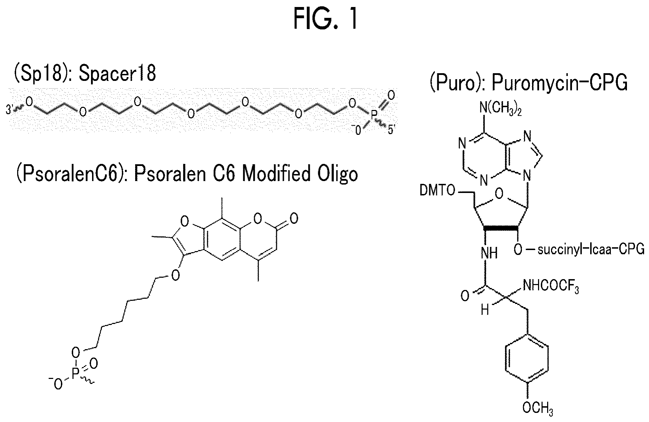

(*See Fig. 1 for the structures of Psoralen C6, Sp18, and Puro. The underlined nucleotides represent that 2'OH of RNA has been formed into a 2'OMe form, and the nucleotides that are not underlined represent unmodified DNA.)

To translate chloroacetylated lysine which is an unnatural amino acid, a tRNA in which the anticodon was CUA and paired with the UAG codon of mRNA was prepared by transcribing the DNA of SEQ ID NO: 18. This tRNA was aminoacylated with an N-chloroacetylated lysine phospho 2'deoxyribocytidylylriboadenosine (pdCpA) ester. This aminoacyl tRNA is referred to as an Aminoacyl tRNA (1).

SEQ ID NO: 18:

GTTGTAAAACGACGGCCAGTGCCAAGCTTGGGCTAATACGACTCACTATAGGGAG
AGTAGTTCAATGGTAGAACGTCGGTCTCTAAAACCGAGCGTTGAGGGTTCGATTC
CTTTCTCTCCCAC

The complex of the library transcription and the puromycin linker were translated in a translation solution including PUREfrex 2.0 (GeneFrontier, PF201-0.25-5) and aminoacyl tRNA. 5.25 µL of the complex, 7.5 µL of PUREfrex 2.0 Solution I, 0.75 µL of Solution II, 1.5 µL of Solution III, and a dried product of Aminoacyl tRNA (1) (final concentration: 0.5 µg/µL) were mixed and reacted at 37°C for 60 minutes to produce mRNA-cyclic peptide conjugate.

15 µL of the translation product and the DNA (final concentration: 10 µmol/L) represented by SEQ ID NO: 19 were mixed in the presence of ReverTra Ace (TOYOBO, TRT-101) (reaction volume of 37.5 µL) and reacted at 37°C for 30 minutes to perform reverse transcription and to produce a cDNA-mRNA-cyclic peptide conjugate.
SEQ ID NO: 19: GCTACCGCCAGAACCACC

Next, 22.5 µL of the reverse transcription product was mixed with 10 µL of the magnetic beads-immobilized IgG1 Fc, and a TBS buffer (20 mmol/L Tris, 150 mmol/L NaCl, 0.1% BSA, 0.05% Tween 20, pH 7.4) (reaction volume: 100 µL), reacted at room temperature for 45 minutes, and the supernatant was recovered to remove the cDNA-mRNA-cyclic peptide conjugate bound to the magnetic beads.

Next, the supernatant was mixed with 10 µL of magnetic beads-immobilized FGFR (reaction volume: 50 µL), and reacted at room temperature for 45 minutes, and then the magnetic beads were washed three times with 100 µL of a TBS buffer to extract a cDNA-mRNA-cyclic peptide conjugate bound to FGFR. The extracted cDNA-mRNA-cyclic peptide conjugate was amplified by the following two-step PCR.

In the first PCR step, an amplification product was obtained by mixing 25 µL of the cDNA-mRNA-polypeptide conjugate with the DNA set forth in SEQ ID NO: 20 (final concentration of 0.5 µmol/L) and the DNA set forth in SEQ ID NO: 21 (final concentration of 0.5 µmol/L) (reaction volume of 50 µL), repeatedly performing, after 98°C/30 seconds, three steps of 98°C/10 seconds, 60°C/10 seconds, and 72°C/10 seconds for 6 to 15 cycles in the presence of Platinum^{™} SuperFi II DNA Polymerase (Thermo, 12361010), and finally performing the treatment of 72°C/5 minutes. The amplification product was purified and diluted to 20 nmol/L.
SEQ ID NO: 20: GGAGATATACATATGGTTAAGAAAACAAAAAC
SEQ ID NO: 21: CTGCTACCGCCAGAACCACC

In the second PCR step, DNA having the same sequence as the original library excluding the random sequence was obtained by mixing the amplification product of the first PCR step (final concentration: 10 nmol/L) with the DNA set forth in SEQ ID NO: 2 (final concentration: 0.5 µmol/L) and the DNA set forth in SEQ ID NO: 3 (final concentration: 0.5 µmol/L), repeatedly performing, after 98°C/30 seconds, three steps of 98°C/10 seconds, 60°C/10 seconds, and 72°C/10 seconds for 6 cycles in the presence of Platinum^{™} SuperFi II DNA Polymerase (Thermo, 12361010), and finally performing the treatment of 72°C/5 minutes. The produced library was purified, diluted to 2 ng/µL and used in the next round.

The base sequence product of the eighth round in the first PCR step was identified according to the standard protocol of Illumina, Inc. using MiSeq (manufactured by Illumina, Inc.) and Miseq Reagent kit v2 (300 cycles) (Illumina, MS-102-2022). As a result of analyzing the identified cyclic peptide group, a large number of cyclic peptides having the following amino acid sequence were found.
X1-Xm-X2-Xn-X3
X1: a glutamine residue or a histidine residue
Xm: a peptide residue containing any 2 to 4 amino acid residues
X2: an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue
Xn: an amino acid residue or a peptide residue containing any 1 to 3 amino acid residues
X3: a phenylalanine residue, a tryptophan residue, a tyrosine residue, or a histidine residue
A part of the cyclic peptides included in the identified cyclic peptide group and a cyclic peptide in which a part of amino acid residues of the cyclic peptides included in the identified cyclic peptide group were substituted, deleted, or inserted were evaluated in the binding affinity evaluation of Example 2.

### <Example 2: Evaluation of binding affinity of peptide (enzyme synthesis product)>

The binding affinity of the peptide to FGFR was evaluated using an ELISA method.

The peptide used for the evaluation was enzymatically synthesized by the following method.

First, a fusion peptide (SEQ ID NO: 25) in which a translation enhancing sequence (SEQ ID NO: 22), a peptide, a Myc tag (SEQ ID NO: 23), and a HiBiT tag (SEQ ID NO: 24) were linked in this order was designed. Next, a template DNA sequence (SEQ ID NO: 26) was designed in which a T7 promoter sequence, a Shine-Dalgarno sequence, a sequence necessary for transcription and translation initiation, such as a start codon (ATG), was provided on the 5' terminal side of the DNA sequence encoding the fused peptide, and a stop codon group was provided on the 3' terminal side thereof. In a case where the triplet TAG codon is included in the template DNA sequence, the above-described codon is assigned to the codon of chloroacetylated lysine, and thus, the peptide encoded by the above-described DNA sequence is a cyclic peptide in which a thiol group of cysteine and a chloroacetyl group of chloroacetylated lysine spontaneously form a thioether bond.
SEQ ID NO: 22: VKKTKT
SEQ ID NO: 23: EQKLISEEDL
SEQ ID NO: 24: VSGWRLFKKIS
SEQ ID NO: 25: MVKKTKT[HCSYERLQFHGHEAPFRVXV]GSGSGSEQKLISEEDLGGSVSGWRLFKKIS
(X is chloroacetylated lysine, [ ] is an example of an amino acid sequence of a peptide to be evaluated, and [ ] is different for each peptide.)
SEQ ID NO: 26: 
GAAATTAATACGACTCACTATAGGGAGACCACAACGGTTTCCCTCTAGAAATAAT
TTTGTTTAACTTTAAGAAGGAGATATACCAATGGTTAAAAAAACAAAAACA[CATT
GCTCTTACGAACGTCTGCAGTTCCATGGTCATGAAGCTCCGTTCCGTGTTTAGGTT]
GGTTCTGGCAGTGGTTCCGAACAGAAACTGATCAGCGAAGAAGATCTGGGTGGCT
CTGTAAGTGGATGGCGATTATTCAAGAAGATTAGCTAATGAATAACTAATCC
([ ] represents an example of a DNA sequence encoding a peptide to be evaluated, and [ ] is different for each peptide.)

The template DNA was produced by a two-step overlap extension PCR.

In the first stage of PCR, the DNA set forth in SEQ ID NO: 29 and the DNA set forth in SEQ ID NO: 30 were linked by mixing four types of DNA, the DNA set forth in SEQ ID NO: 27, the DNA set forth in SEQ ID NO: 28, the DNA set forth in SEQ ID NO: 29, and the DNA set forth in SEQ ID NO: 30, such that the concentrations thereof were 0.3 µmol/L, 0.3 µmol/L, 0.05 µmol/L, and 0.05 µmol/L, and in the presence of PrimeSTAR Max (TaKaRa, R045B), after performing a step of 98°C/10 seconds, 39°C/5 seconds, and 72°C/5 seconds, repeatedly performing three steps of 98°C/10 seconds, 58°C/5 seconds, and 72°C/5 seconds for 27 cycles. The linked DNA was purified and diluted to 50 ng/µL.
SEQ ID NO: 27: GAAATTAATACGACTCACTATAGG
SEQ ID NO: 28: GAACCACTGCCAGAACC
SEQ ID NO: 29: 
GAAATTAATACGACTCACTATAGGGAGACCACAACGGTTTCCCTCTAGAAATAAT
TTTGTTTAACTTTAAGAAGGAGATATACCAATGGTTAAAAAAACAAAAAC
SEQ ID NO: 30: 
GAACCACTGCCAGAACC[AACCTAAACACGGAACGGAGCTTCATGACCATGGAAC
TGCAGACGTTCGTAAGAGCAATG]TGTTTTTGTTTTTTTAAC
([ ] represents an example of a DNA sequence encoding a peptide to be evaluated, and [ ] is different for each peptide.)

In the second stage of PCR, by mixing four types of DNA, the DNA set forth in SEQ ID NO: 27, the DNA set forth in SEQ ID NO: 31, the DNA set forth in SEQ ID NO: 32, and the purified product of the first stage, such that the concentrations thereof were 0.3 µmol/L, 0.3 µmol/L, 0.0025 µmol/L, and 0.4 ng/µL, and in the presence of PrimeSTAR Max (TaKaRa, R045B), and repeatedly performing three steps of 98°C/10 seconds, 58°C/5 seconds, and 72°C/5 seconds for 30 cycles, the DNA set forth in SEQ ID NO: 32 and the purified product of the first stage were linked to obtain the template DNA (SEQ ID NO: 25). The template DNA was purified and diluted to 50 ng/µL.
SEQ ID NO: 31: GGATTAGTTATTCATTAGCTAATC
SEQ ID NO: 32:

GGTTCTGGCAGTGGTTCCGAACAGAAACTGATCAGCGAAGAAGATCTGGGTGGCT
CTGTAAGTGGATGGCGATTATTCAAGAAGATTAGCTAATGAATAACTAATCC

The cell-free enzymatic synthesis of the polypeptide was performed in a translation solution containing a template DNA, PUREfrex 2.0 (GeneFrontier, PF201-0.25-5), and Aminoacyl tRNA (1). 1.75 µL (62.5 ng) of the template DNA prepared to 50 ng/µL, 2.5 µL of PUREfrex 2.0 Solution I, 0.25 µL of Solution II, 0.5 µL of Solution III, and a dried product of Aminoacyl tRNA (1) (final concentration: 0.5 ng/µL) were mixed and mixture was subjected to a reaction at 37°C for 1 hour.

The concentration of the enzymatically synthesized peptide was measured using the peptide diluted with Can Get Signal Immunoreaction Enhancer Solution I (TOYOBO, NKB-101), Nano-Glo HiBiT Lytic Detection System (Promega, N3040), and a chemical synthetic peptide for a calibration curve having a known concentration (SEQ ID NO: 33) according to the standard protocol of the Nano-Glo HiBiT Lytic Detection System.
SEQ ID NO: 33: EQKLISEEDLGGSVSGWRLFKKIS

The binding affinity of the enzymatically synthesized peptide to FGFR was measured by evaluating the amount of the peptide bound to FGFR by ELISA.

First, 5 ng of Recombinant Human FGFR2 alpha (IIIc) Fc Chimera Protein (R&D systems, 712-FR-050) was immobilized in each well of a 96-well plate, and blocked with Pierce Protein-Free (PBS) Blocking Buffer (Thermo, 37572), and a peptide diluted with Can Get Signal Immunoreaction Enhancer Solution I (TOYOBO, NKB-101) was then added and reacted at room temperature for 3 hours. After washing with 0.05% Tween 20-containing phosphate buffered saline (PBS), an anti-Myc antibody (Cell Signaling, 14038S) diluted with Can Get Signal Immunoreaction Enhancer Solution II (TOYOBO, NKB-101) was added and reacted at room temperature for 2 hours. After washing with 0.05% Tween 20-containing PBS, SuperSignal ELISA Femto Substrate (Thermo, 37075) was added, and the luminescence amount was measured. The dose-response relationship was calculated from using the difference between the luminescence amount in the well in which FGFR was immobilized and the luminescence amount in the well in which FGFR was not immobilized, and the concentration of the peptide at the time of reaction, and the binding affinity to FGFR was evaluated according to the following evaluation criteria. The evaluation result preferably satisfies the evaluation criteria A, B, or C.

### (Evaluation criteria for FGFR binding affinity)

In a case where a value of a luminescence signal emitted by the cyclic peptide set forth in SEQ ID NO: 47 at a reaction concentration of 10 nmol/L is standardized to 1, evaluation criteria of the luminescence signal value emitted by the cyclic peptide at a reaction concentration of 10 nmol/L is as follows.
A: 0.7 or more
B: 0.5 or more and less than 0.7
C: 0.1 or more and less than 0.5
D: less than 0.1.

The evaluation results of the cyclic peptide are shown in Table 4.

The amino acid residues described in parentheses indicate amino acid residues constituting a cyclized structure by a thioether bond or a disulfide bond between side chains. For example, K(acetyl) refers to a structure in which a lysine residue side chain is modified with a haloacetyl group, and the cyclic peptide has a cyclized structure in which a halogen atom of the haloacetyl group is substituted with a thiol group of another residue.

SEQ ID NO: 34 is a cyclic peptide reported in WO2000/003245A, and SEQ ID NO: 36 is a cyclic peptide obtained by converting SEQ ID NO: 34 into a thioether-type cyclized structure.

SEQ ID NO: 35 is a linear peptide reported in Dev. Neurobiol. (2009) 69 (13): 837-854 (Agonists of fibroblast growth factor receptor induce neurite outgrowth and survival of cerebellar granule neurons).

SEQ ID NO: 37 and subsequent sequence numbers are cyclic peptides to be targeted by the present invention.

**[Table 4]**

| SEQ ID NO: | Sequence | Evaluation result of binding affinity to FGFR |
|---|---|---|
| 34 | (C)-RALLRGAPFHLAE-(C) | D |
| 35 | YRSRKYSSWYVALKR | D |
| 36 | (C)-RALLRGAPFHLAE-(K(acetyl)) | D |
| 37 | Q-(C)-QLNYWDDLQFTYYLQY-(K(acetyl))-V | A |
| 38 | Q-(C)-SYLYQYVFERYKVTVP-(K(acetyl))-D | A |
| 39 | Q-(C)-ENWLSQDQWKYFWQY-(K(acetyl))-I | A |
| 40 | D-(C)-SGNFFSKEQFHYLELF-(K(acetyl))-K | A |
| 41 | L-(C)-GNYITPLQWKYFQTY-(K(acetyl))-W | A |
| 42 | Q-(C)-YFLSQYILERYKVH-(K(acetyl))-L | A |
| 43 | H-(C)-NANFVYQQQYLYLLQY-(K(acetyl))-L | B |
| 44 | A-(C)-EYNYWNQVQFTFLIAF-(K(acetyl))-R | B |
| 45 | T-(C)-PQNFRDELQFTYFTQY-(K(acetyl))-Y | A |
| 46 | S-(C)-IQQNYFWQYVFERYRY-(K(acetyl))-Q | B |
| 47 | K-(C)-PQNFINPVQWRYFQHY-(K(acetyl))-N | A |
| 48 | G-(C)-YWNEVQYYYLQQY-(K(acetyl))-Q | A |
| 49 | s-(C)-QGNWISNTQFRYFIAY-(K(acetyl))-T | A |
| 50 | A-(C)-GQNFYHPVQFHYFIAY-(K(acetyl))-Q | A |
| 51 | V-(C)-HSNFLSAVQYAYFYQY-(K(acetyl))-H | A |
| 52 | V-(C)-NENNWSRSQYHFFLQY-(K(acetyl))-Y | C |
| 53 | A-(C)-PQNFINPVQWRYFQHY-(K(acetyl))-N | A |
| 54 | K-(C)-AQNFINPVQWRYFQHY-(K(acetyl))-N | A |
| 55 | K-(C)-PANFINPVQWRYFQHY-(K(acetyl))-N | A |
| 56 | K-(C)-PQAFINPVQWRYFQHY-(K(acetyl))-N | C |
| 57 | K-(C)-PQNAINPVQWRYFQHY-(K(acetyl))-N | B |
| 58 | K-(C)-PQNFANPVQWRYFQHY-(K(acetyl))-N | B |
| 59 | K-(C)-PQNFIAPVQWRYFQHY-(K(acetyl))-N | B |
| 60 | K-(C)-PQNFINAVQWRYFQHY-(K(acetyl))-N | A |
| 61 | K-(C)-PQNFINPAQWRYFQHY-(K(acetyl))-N | A |
| 62 | K-(C)-PQNFINPVQARYFQHY-(K(acetyl))-N | C |
| 63 | K-(C)-PQNFINPVQWAYFQHY-(K(acetyl))-N | A |
| 64 | K-(C)-PQNFINPVQWRAFQHY-(K(acetyl))-N | B |
| 65 | K-(C)-PQNFINPVQWRYFAHY-(K(acetyl))-N | A |
| 66 | K-(C)-PQNFINPVQWRYFQAY-(K(acetyl))-N | A |
| 67 | K-(C)-PQNFINPVQWRYFQHY-(K(acetyl))-A | A |
| 68 | K-(C)-QNFINPVQWRYFQHY-(K(acetyl))-N | A |
| 69 | K-(C)-PQNFINPVQWRYFQHYA-(K(acetyl))-N | C |
| 70 | K-(C)-APQNFINPVQWRYFQHY-(K(acetyl))-N | B |
| 71 | G-(C)-PQNFINPVQWRYFQHY-(K(acetyl))-N | A |
| 72 | P-(C)-PQNFINPVQWRYFQHY-(K(acetyl))-N | A |
| 73 | L-(C)-PQNFINPVQWRYFQHY-(K(acetyl))-N | A |
| 74 | I-(C)-PQNFINPVQWRYFQHY-(K(acetyl))-N | A |
| 75 | V-(C)-PQNFINPVQWRYFQHY-(K(acetyl))-N | A |
| 76 | F-(C)-PQNFINPVQWRYFQHY-(K(acetyl))-N | A |
| 77 | Y-(C)-PQNFINPVQWRYFQHY-(K(acetyl))-N | A |
| 78 | W-(C)-PQNFINPVQWRYFQHY-(K(acetyl))-N | A |
| 79 | N-(C)-PQNFINPVQWRYFQHY-(K(acetyl))-N | A |
| 80 | Q-(C)-PQNFINPVQWRYFQHY-(K(acetyl))-N | A |
| 81 | S-(C)-PQNFINPVQWRYFQHY-(K(acetyl))-N | A |
| 82 | T-(C)-PQNFINPVQWRYFQHY-(K(acetyl))-N | A |
| 83 | D-(C)-PQNFINPVQWRYFQHY-(K(acetyl))-N | A |
| 84 | E-(C)-PQNFINPVQWRYFQHY-(K(acetyl))-N | A |
| 85 | R-(C)-PQNFINPVQWRYFQHY-(K(acetyl))-N | A |
| 86 | H-(C)-PQNFINPVQWRYFQHY-(K(acetyl))-N | A |
| 87 | Q-(C)-PQNFINPVHWRYFQHY-(K(acetyl))-N | A |
| 88 | Q-(C)-PQNFINPVQWRYLQHY-(K(acetyl))-N | A |
| 89 | Q-(C)-PQNFINPVQWRYYQHY-(K(acetyl))-N | B |
| 90 | Q-(C)-PQNFINPVQWRYRQHY-(K(acetyl))-N | C |
| 91 | Q-(C)-PQNFINPVQWRYFQHV-(K(acetyl))-N | C |
| 92 | Q-(C)-PQNFINPVQWRYFQHF-(K(acetyl))-N | A |
| 93 | Q-(C)-PQNFINPVQWRYFQHW-(K(acetyl))-N | A |
| 94 | Q-(C)-PQNFINPVQWRYFQHS-(K(acetyl))-N | C |
| 95 | Q-(C)-PQGFINPVQWRYFQHY-(K(acetyl))-N | C |
| 96 | Q-(C)-PQYFINPVQWRYFQHY-(K(acetyl))-N | C |
| 97 | Q-(C)-PQSFINPVQWRYFQHY-(K(acetyl))-N | C |
| 98 | Q-(C)-PQTFINPVQWRYFQHY-(K(acetyl))-N | C |
| 99 | Q-(C)-PQEFINPVQWRYFQHY-(K(acetyl))-N | C |
| 100 | Q-(C)-PQHFINPVQWRYFQHY-(K(acetyl))-N | C |
| 101 | Q-(C)-PQNFINPVQIRYFQHY-(K(acetyl))-N | C |
| 102 | Q-(C)-PQNFINPVQVRYFQHY-(K(acetyl))-N | C |
| 103 | Q-(C)-PQNFINPVQFRYFQHY-(K(acetyl))-N | B |
| 104 | Q-(C)-PQNFINPVQYRYFQHY-(K(acetyl))-N | B |
| 105 | Q-(C)-PQNFINPVQSRYFQHY-(K(acetyl))-N | C |
| 106 | Q-(C)-PQNFINPVQHRYFQHY-(K(acetyl))-N | A |
| 107 | Y-(C)-TDNYIDDIQFWYLFLY-(K(acetyl))-Q | B |
| 108 | L-(C)-RNYINPQQWRYFALY-(K(acetyl))-Y | A |
| 109 | D-(C)-FINELQYFYIQQY-(K(acetyl))-K | B |
| 110 | Q-(C)-QTDHQQQLYFIYQYLQ-(K(acetyl))-I | C |
| 111 | Q-(C)-DQEFYLALYAWTLRSY-(K(acetyl))-L | C |
| 112 | H-(C)-QPEYELYYIYLYLNAL-(K(acetyl))-I | C |
| 113 | R-(C)-INYWSGQQFYFFQQY-(K(acetyl))-F | C |
| 114 | Y-(C)-TDNYIDDIQFWYLFLY-(K(acetyl))-H | A |
| 115 | N-(C)-PNYWSQIQWHYFQAY-(K(acetyl))-Q | A |
| 116 | Q-(C)-YQNWYLYQYLVERYY-(K(acetyl))-D | C |
| 117 | L-(C)-RNYINPQQWRYFALY-(K(acetyl))-N | A |
| 118 | L-(C)-PVPQQTDYLRWYQIIH(K(acetyl))-Q | C |
| 119 | Q-(C)-RYFWQYVLERYRVRLP-(K(acetyl))-E | A |
| 120 | Q-(C)-WYIQQYIWERYFLWHN-(K(acetyl))-E | C |
| 121 | D-(C)-YWDDQQYSFLQLY-(K(acetyl))-R | A |
| 122 | Q-(C)-ENWVNSTQFEFFWQY-(K(acetyl))-T | B |
| 123 | Q-(C)-QNFITEVQFTYFWHY-(K(acetyl))-T | A |
| 124 | D-(C)-RDLQLFYIYQYLDAL-(K(acetyl))-K | C |
| 125 | Y-(C)-RDNYFYLEQWQYLYLY-(K(acetyl))-H | A |
| 126 | L-(C)-EINWLSQEQYGYFIEY-(K(acetyl))-T | A |
| 127 | Y-(C)-PYNFYNIIQFHYFLTY-(K(acetyl))-T | A |
| 128 | P-(C)-VQNYFNDLQWIFLQHY-(K(acetyl))-R | A |
| 129 | E-(C)-PWNFVNQLQWQYFITY-(K(acetyl))-V | A |
| 130 | H-(C)-DGNWWNSQQFFYLLLY-(K(acetyl))-H | A |
| 131 | Y-(C)-EENYLDPEQYQYFIYF-(K(acetyl))-Y | A |
| 132 | E-(C)-QENFVDRQQYWYFLSY-(K(acetyl))-F | A |
| 133 | E-(C)-LGNYFSHEQYQYLLQY-(K(acetyl))-I | A |
| 134 | L-(C)-ADQYDYLLQYVFWRTY-(K(acetyl))-I | C |
| 135 | Q-(C)-TYLAQYLVERYHLT-(K(acetyl))-T | C |
| 136 | G-(C)-EENYLDPEQYQYFIYF-(K(acetyl))-Y | A |
| 137 | P-(C)-EENYLDPEQYQYFIYF-(K(acetyl))-Y | A |
| 138 | L-(C)-EENYLDPEQYQYFIYF-(K(acetyl))-Y | A |
| 139 | I-(C)-EENYLDPEQYQYFIYF-(K(acetyl))-Y | A |
| 140 | V-(C)-EENYLDPEQYQYFIYF-(K(acetyl))-Y | A |
| 141 | F-(C)-EENYLDPEQYQYFIYF-(K(acetyl))-Y | A |
| 142 | W-(C)-EENYLDPEQYQYFIYF-(K(acetyl))-Y | A |
| 143 | N-(C)-EENYLDPEQYQYFIYF-(K(acetyl))-Y | A |
| 144 | Q-(C)-EENYLDPEQYQYFIYF-(K(acetyl))-Y | A |
| 145 | S-(C)-EENYLDPEQYQYFIYF-(K(acetyl))-Y | A |
| 146 | T-(C)-EENYLDPEQYQYFIYF-(K(acetyl))-Y | A |
| 147 | D-(C)-EENYLDPEQYQYFIYF-(K(acetyl))-Y | A |
| 148 | E-(C)-EENYLDPEQYQYFIYF-(K(acetyl))-Y | A |
| 149 | R-(C)-EENYLDPEQYQYFIYF-(K(acetyl))-Y | A |
| 150 | H-(C)-EENYLDPEQYQYFIYF-(K(acetyl))-Y | A |
| 151 | Y-(C)-GENYLDPEQYQYFIYF-(K(acetyl))-Y | A |
| 152 | Y-(C)-PENYLDPEQYQYFIYF-(K(acetyl))-Y | A |
| 153 | Y-(C)-LENYLDPEQYQYFIYF-(K(acetyl))-Y | A |
| 154 | Y-(C)-IENYLDPEQYQYFIYF-(K(acetyl))-Y | A |
| 155 | Y-(C)-VENYLDPEQYQYFIYF-(K(acetyl))-Y | A |
| 156 | Y-(C)-FENYLDPEQYQYFIYF-(K(acetyl))-Y | A |
| 157 | Y-(C)-YENYLDPEQYQYFIYF-(K(acetyl))-Y | A |
| 158 | Y-(C)-WENYLDPEQYQYFIYF-(K(acetyl))-Y | A |
| 159 | Y-(C)-NENYLDPEQYQYFIYF-(K(acetyl))-Y | A |
| 160 | Y-(C)-QENYLDPEQYQYFIYF-(K(acetyl))-Y | A |
| 161 | Y-(C)-SENYLDPEQYQYFIYF-(K(acetyl))-Y | A |
| 162 | Y-(C)-TENYLDPEQYQYFIYF-(K(acetyl))-Y | A |
| 163 | Y-(C)-DENYLDPEQYQYFIYF-(K(acetyl))-Y | A |
| 164 | Y-(C)-RENYLDPEQYQYFIYF-(K(acetyl))-Y | A |
| 165 | Y-(C)-HENYLDPEQYQYFIYF-(K(acetyl))-Y | A |
| 166 | Y-(C)-EGNYLDPEQYQYFIYF-(K(acetyl))-Y | A |
| 167 | Y-(C)-EPNYLDPEQYQYFIYF-(K(acetyl))-Y | A |
| 168 | Y-(C)-ELNYLDPEQYQYFIYF-(K(acetyl))-Y | A |
| 169 | Y-(C)-EINYLDPEQYQYFIYF-(K(acetyl))-Y | A |
| 170 | Y-(C)-EVNYLDPEQYQYFIYF-(K(acetyl))-Y | A |
| 171 | Y-(C)-EFNYLDPEQYQYFIYF-(K(acetyl))-Y | B |
| 172 | Y-(C)-EYNYLDPEQYQYFIYF-(K(acetyl))-Y | A |
| 173 | Y-(C)-EWNYLDPEQYQYFIYF-(K(acetyl))-Y | A |
| 174 | Y-(C)-ENNYLDPEQYQYFIYF-(K(acetyl))-Y | A |
| 175 | Y-(C)-EQNYLDPEQYQYFIYF-(K(acetyl))-Y | A |
| 176 | Y-(C)-ESNYLDPEQYQYFIYF-(K(acetyl))-Y | A |
| 177 | Y-(C)-ETNYLDPEQYQYFIYF-(K(acetyl))-Y | A |
| 178 | Y-(C)-EDNYLDPEQYQYFIYF-(K(acetyl))-Y | A |
| 179 | Y-(C)-ERNYLDPEQYQYFIYF-(K(acetyl))-Y | A |
| 180 | Y-(C)-EHNYLDPEQYQYFIYF-(K(acetyl))-Y | A |
| 181 | Y-(C)-EEGYLDPEQYQYFIYF-(K(acetyl))-Y | B |
| 182 | Y-(C)-EEWYLDPEQYQYFIYF-(K(acetyl))-Y | C |
| 183 | Y-(C)-EEQYLDPEQYQYFIYF-(K(acetyl))-Y | C |
| 184 | Y-(C)-EESYLDPEQYQYFIYF-(K(acetyl))-Y | C |
| 185 | Y-(C)-EETYLDPEQYQYFIYF-(K(acetyl))-Y | C |
| 186 | Y-(C)-EENLLDPEQYQYFIYF-(K(acetyl))-Y | C |
| 187 | Y-(C)-EENFLDPEQYQYFIYF-(K(acetyl))-Y | A |
| 188 | Y-(C)-EENWLDPEQYQYFIYF-(K(acetyl))-Y | A |
| 189 | Y-(C)-EENNLDPEQYQYFIYF-(K(acetyl))-Y | B |
| 190 | Y-(C)-EENQLDPEQYQYFIYF-(K(acetyl))-Y | C |
| 191 | Y-(C)-EENSLDPEQYQYFIYF-(K(acetyl))-Y | C |
| 192 | Y-(C)-EENDLDPEQYQYFIYF-(K(acetyl))-Y | C |
| 193 | Y-(C)-EENRLDPEQYQYFIYF-(K(acetyl))-Y | C |
| 194 | Y-(C)-EENHLDPEQYQYFIYF-(K(acetyl))-Y | A |
| 195 | Y-(C)-EENYIDPEQYQYFIYF-(K(acetyl))-Y | A |
| 196 | Y-(C)-EENYVDPEQYQYFIYF-(K(acetyl))-Y | A |
| 197 | Y-(C)-EENYFDPEQYQYFIYF-(K(acetyl))-Y | A |
| 198 | Y-(C)-EENYYDPEQYQYFIYF-(K(acetyl))-Y | A |
| 199 | Y-(C)-EENYWDPEQYQYFIYF-(K(acetyl))-Y | A |
| 200 | Y-(C)-EENYNDPEQYQYFIYF-(K(acetyl))-Y | C |
| 201 | Y-(C)-EENYQDPEQYQYFIYF-(K(acetyl))-Y | A |
| 202 | Y-(C)-EENYSDPEQYQYFIYF-(K(acetyl))-Y | B |
| 203 | Y-(C)-EENYTDPEQYQYFIYF-(K(acetyl))-Y | A |
| 204 | Y-(C)-EENYEDPEQYQYFIYF-(K(acetyl))-Y | C |
| 205 | Y-(C)-EENYRDPEQYQYFIYF-(K(acetyl))-Y | A |
| 206 | Y-(C)-EENYHDPEQYQYFIYF-(K(acetyl))-Y | A |
| 207 | Y-(C)-EENYLGPEQYQYFIYF-(K(acetyl))-Y | A |
| 208 | Y-(C)-EENYLLPEQYQYFIYF-(K(acetyl))-Y | C |
| 209 | Y-(C)-EENYLIPEQYQYFIYF-(K(acetyl))-Y | C |
| 210 | Y-(C)-EENYLVPEQYQYFIYF-(K(acetyl))-Y | C |
| 211 | Y-(C)-EENYLFPEQYQYFIYF-(K(acetyl))-Y | A |
| 212 | Y-(C)-EENYLYPEQYQYFIYF-(K(acetyl))-Y | A |
| 213 | Y-(C)-EENYLWPEQYQYFIYF-(K(acetyl))-Y | B |
| 214 | Y-(C)-EENYLNPEQYQYFIYF-(K(acetyl))-Y | A |
| 215 | Y-(C)-EENYLQPEQYQYFIYF-(K(acetyl))-Y | A |
| 216 | Y-(C)-EENYLSPEQYQYFIYF-(K(acetyl))-Y | A |
| 217 | Y-(C)-EENYLTPEQYQYFIYF-(K(acetyl))-Y | A |
| 218 | Y-(C)-EENYLEPEQYQYFIYF-(K(acetyl))-Y | A |
| 219 | Y-(C)-EENYLRPEQYQYFIYF-(K(acetyl))-Y | C |
| 220 | Y-(C)-EENYLHPEQYQYFIYF-(K(acetyl))-Y | A |
| 221 | Y-(C)-EENYLDGEQYQYFIYF-(K(acetyl))-Y | A |
| 222 | Y-(C)-EENYLDLEQYQYFIYF-(K(acetyl))-Y | A |
| 223 | Y-(C)-EENYLDIEQYQYFIYF-(K(acetyl))-Y | A |
| 224 | Y-(C)-EENYLDVEQYQYFIYF-(K(acetyl))-Y | A |
| 225 | Y-(C)-EENYLDFEQYQYFIYF-(K(acetyl))-Y | A |
| 226 | Y-(C)-EENYLDYEQYQYFIYF-(K(acetyl))-Y | A |
| 227 | Y-(C)-EENYLDWEQYQYFIYF-(K(acetyl))-Y | A |
| 228 | Y-(C)-EENYLDNEQYQYFIYF-(K(acetyl))-Y | A |
| 229 | Y-(C)-EENYLDQEQYQYFIYF-(K(acetyl))-Y | A |
| 230 | Y-(C)-EENYLDSEQYQYFIYF-(K(acetyl))-Y | A |
| 231 | Y-(C)-EENYLDTEQYQYFIYF-(K(acetyl))-Y | A |
| 232 | Y-(C)-EENYLDDEQYQYFIYF-(K(acetyl))-Y | A |
| 233 | Y-(C)-EENYLDEEQYQYFIYF-(K(acetyl))-Y | A |
| 234 | Y-(C)-EENYLDREQYQYFIYF-(K(acetyl))-Y | A |
| 235 | Y-(C)-EENYLDHEQYQYFIYF-(K(acetyl))-Y | A |
| 236 | Y-(C)-EENYLDPGQYQYFIYF-(K(acetyl))-Y | C |
| 237 | Y-(C)-EENYLDPPQYQYFIYF-(K(acetyl))-Y | A |
| 238 | Y-(C)-EENYLDPLQYQYFIYF-(K(acetyl))-Y | A |
| 239 | Y-(C)-EENYLDPIQYQYFIYF-(K(acetyl))-Y | A |
| 240 | Y-(C)-EENYLDPVQYQYFIYF-(K(acetyl))-Y | A |
| 241 | Y-(C)-EENYLDPFQYQYFIYF-(K(acetyl))-Y | A |
| 242 | Y-(C)-EENYLDPYQYQYFIYF-(K(acetyl))-Y | A |
| 243 | Y-(C)-EENYLDPWQYQYFIYF-(K(acetyl))-Y | A |
| 244 | Y-(C)-EENYLDPNQYQYFIYF-(K(acetyl))-Y | A |
| 245 | Y-(C)-EENYLDPQQYQYFIYF-(K(acetyl))-Y | A |
| 246 | Y-(C)-EENYLDPSQYQYFIYF-(K(acetyl))-Y | A |
| 247 | Y-(C)-EENYLDPTQYQYFIYF-(K(acetyl))-Y | A |
| 248 | Y-(C)-EENYLDPDQYQYFIYF-(K(acetyl))-Y | A |
| 249 | Y-(C)-EENYLDPRQYQYFIYF-(K(acetyl))-Y | A |
| 250 | Y-(C)-EENYLDPHQYQYFIYF-(K(acetyl))-Y | A |
| 251 | Y-(C)-EENYLDPEHYQYFIYF-(K(acetyl))-Y | C |
| 252 | Y-(C)-EENYLDPEQLQYFIYF-(K(acetyl))-Y | C |
| 253 | Y-(C)-EENYLDPEQIQYFIYF-(K(acetyl))-Y | C |
| 254 | Y-(C)-EENYLDPEQFQYFIYF-(K(acetyl))-Y | A |
| 255 | Y-(C)-EENYLDPEQWQYFIYF-(K(acetyl))-Y | A |
| 256 | Y-(C)-EENYLDPEQHQYFIYF-(K(acetyl))-Y | A |
| 257 | Y-(C)-EENYLDPEQYGYFIYF-(K(acetyl))-Y | A |
| 258 | Y-(C)-EENYLDPEQYLYFIYF-(K(acetyl))-Y | A |
| 259 | Y-(C)-EENYLDPEQYIYFIYF-(K(acetyl))-Y | A |
| 260 | Y-(C)-EENYLDPEQYVYFIYF-(K(acetyl))-Y | A |
| 261 | Y-(C)-EENYLDPEQYFYFIYF-(K(acetyl))-Y | B |
| 262 | Y-(C)-EENYLDPEQYYYFIYF-(K(acetyl))-Y | A |
| 263 | Y-(C)-EENYLDPEQYWYFIYF-(K(acetyl))-Y | A |
| 264 | Y-(C)-EENYLDPEQYNYFIYF-(K(acetyl))-Y | A |
| 265 | Y-(C)-EENYLDPEQYSYFIYF-(K(acetyl))-Y | A |
| 266 | Y-(C)-EENYLDPEQYTYFIYF-(K(acetyl))-Y | A |
| 267 | Y-(C)-EENYLDPEQYDYFIYF-(K(acetyl))-Y | A |
| 268 | Y-(C)-EENYLDPEQYEYFIYF-(K(acetyl))-Y | A |
| 269 | Y-(C)-EENYLDPEQYRYFIYF-(K(acetyl))-Y | A |
| 270 | Y-(C)-EENYLDPEQYHYFIYF-(K(acetyl))-Y | A |
| 271 | Y-(C)-EENYLDPEQYQIFIYF-(K(acetyl))-Y | C |
| 272 | Y-(C)-EENYLDPEQYQFFIYF-(K(acetyl))-Y | A |
| 273 | Y-(C)-EENYLDPEQYQNFIYF-(K(acetyl))-Y | C |
| 274 | Y-(C)-EENYLDPEQYQHFIYF-(K(acetyl))-Y | C |
| 275 | Y-(C)-EENYLDPEQYQYLIYF-(K(acetyl))-Y | A |
| 276 | Y-(C)-EENYLDPEQYQYYIYF-(K(acetyl))-Y | B |
| 277 | Y-(C)-EENYLDPEQYQYFGYF-(K(acetyl))-Y | C |
| 278 | Y-(C)-EENYLDPEQYQYFLYF-(K(acetyl))-Y | A |
| 279 | Y-(C)-EENYLDPEQYQYFVYF-(K(acetyl))-Y | A |
| 280 | Y-(C)-EENYLDPEQYQYFFYF-(K(acetyl))-Y | A |
| 281 | Y-(C)-EENYLDPEQYQYFYYF-(K(acetyl))-Y | A |
| 282 | Y-(C)-EENYLDPEQYQYFWYF-(K(acetyl))-Y | A |
| 283 | Y-(C)-EENYLDPEQYQYFNYF-(K(acetyl))-Y | A |
| 284 | Y-(C)-EENYLDPEQYQYFQYF-(K(acetyl))-Y | A |
| 285 | Y-(C)-EENYLDPEQYQYFSYF-(K(acetyl))-Y | A |
| 286 | Y-(C)-EENYLDPEQYQYFTYF-(K(acetyl))-Y | A |
| 287 | Y-(C)-EENYLDPEQYQYFDYF-(K(acetyl))-Y | A |
| 288 | Y-(C)-EENYLDPEQYQYFEYF-(K(acetyl))-Y | A |
| 289 | Y-(C)-EENYLDPEQYQYFRYF-(K(acetyl))-Y | A |
| 290 | Y-(C)-EENYLDPEQYQYFHYF-(K(acetyl))-Y | A |
| 291 | Y-(C)-EENYLDPEQYQYFIGF-(K(acetyl))-Y | B |
| 292 | Y-(C)-EENYLDPEQYQYFIPF-(K(acetyl))-Y | C |
| 293 | Y-(C)-EENYLDPEQYQYFILF-(K(acetyl))-Y | A |
| 294 | Y-(C)-EENYLDPEQYQYFIIF-(K(acetyl))-Y | B |
| 295 | Y-(C)-EENYLDPEQYQYFIVF-(K(acetyl))-Y | A |
| 296 | Y-(C)-EENYLDPEQYQYFIFF-(K(acetyl))-Y | A |
| 297 | Y-(C)-EENYLDPEQYQYFIWF-(K(acetyl))-Y | A |
| 298 | Y-(C)-EENYLDPEQYQYFINF-(K(acetyl))-Y | A |
| 299 | Y-(C)-EENYLDPEQYQYFIQF-(K(acetyl))-Y | A |
| 300 | Y-(C)-EENYLDPEQYQYFISF-(K(acetyl))-Y | A |
| 301 | Y-(C)-EENYLDPEQYQYFITF-(K(acetyl))-Y | A |
| 302 | Y-(C)-EENYLDPEQYQYFIDF-(K(acetyl))-Y | A |
| 303 | Y-(C)-EENYLDPEQYQYFIEF-(K(acetyl))-Y | A |
| 304 | Y-(C)-EENYLDPEQYQYFIRF-(K(acetyl))-Y | A |
| 305 | Y-(C)-EENYLDPEQYQYFIHF-(K(acetyl))-Y | A |
| 306 | Y-(C)-EENYLDPEQYQYFIYY (K(acetyl))-Y | A |
| 307 | Y-(C)-EENYLDPEQYQYFIYW-(K(acetyl))-Y | A |
| 308 | Y-(C)-EENYLDPEQYQYFIYF-(K(acetyl))-G | A |
| 309 | Y-(C)-EENYLDPEQYQYFIYF-(K(acetyl))-L | A |
| 310 | Y-(C)-EENYLDPEQYQYFIYF-(K(acetyl))-I | A |
| 311 | Y-(C)-EENYLDPEQYQYFIYF-(K(acetyl))-V | A |
| 312 | Y-(C)-EENYLDPEQYQYFIYF-(K(acetyl))-F | A |
| 313 | Y-(C)-EENYLDPEQYQYFIYF-(K(acetyl))-W | A |
| 314 | Y-(C)-EENYLDPEQYQYFIYF-(K(acetyl))-N | A |
| 315 | Y-(C)-EENYLDPEQYQYFIYF-(K(acetyl))-Q | A |
| 316 | Y-(C)-EENYLDPEQYQYFIYF-(K(acetyl))-S | A |
| 317 | Y-(C)-EENYLDPEQYQYFIYF-(K(acetyl))-T | A |
| 318 | Y-(C)-EENYLDPEQYQYFIYF-(K(acetyl))-D | A |
| 319 | Y-(C)-EENYLDPEQYQYFIYF-(K(acetyl))-E | A |
| 320 | Y-(C)-EENYLDPEQYQYFIYF-(K(acetyl))-R | A |
| 321 | Y-(C)-EENYLDPEQYQYFIYF-(K(acetyl))-H | A |
| 322 | A-(C)-EENYLDPEQYQYFIYF-(K(acetyl))-Y | A |
| 323 | Y-(C)-AENYLDPEQYQYFIYF-(K(acetyl))-Y | A |
| 324 | Y-(C)-EANYLDPEQYQYFIYF-(K(acetyl))-Y | A |
| 325 | Y-(C)-EEAYLDPEQYQYFIYF-(K(acetyl))-Y | C |
| 326 | Y-(C)-EENALDPEQYQYFIYF-(K(acetyl))-Y | C |
| 327 | Y-(C)-EENYADPEQYQYFIYF-(K(acetyl))-Y | C |
| 328 | Y-(C)-EENYLAPEQYQYFIYF-(K(acetyl))-Y | A |
| 329 | Y-(C)-EENYLDAEQYQYFIYF-(K(acetyl))-Y | A |
| 330 | Y-(C)-EENYLDPAQYQYFIYF-(K(acetyl))-Y | A |
| 331 | Y-(C)-EENYLDPEQYAYFIYF-(K(acetyl))-Y | A |
| 332 | Y-(C)-EENYLDPEQYQYFAYF-(K(acetyl))-Y | A |
| 333 | Y-(C)-EENYLDPEQYQYFIAF-(K(acetyl))-Y | A |
| 334 | Y-(C)-EENYLDPEQYQYFIYF-(K(acetyl))-A | A |
| 335 | K-(C)-EENYLDPEQYQYFIYF-(K(acetyl))-Y | A |
| 336 | Y-(C)-KENYLDPEQYQYFIYF-(K(acetyl))-Y | A |
| 337 | Y-(C)-EKNYLDPEQYQYFIYF-(K(acetyl))-Y | A |
| 338 | Y-(C)-EENKLDPEQYQYFIYF-(K(acetyl))-Y | C |
| 339 | Y-(C)-EENYKDPEQYQYFIYF-(K(acetyl))-Y | A |
| 340 | Y-(C)-EENYLKPEQYQYFIYF-(K(acetyl))-Y | C |
| 341 | Y-(C)-EENYLDKEQYQYFIYF-(K(acetyl))-Y | A |
| 342 | Y-(C)-EENYLDPKQYQYFIYF-(K(acetyl))-Y | A |
| 343 | Y-(C)-EENYLDPEQYKYFIYF-(K(acetyl))-Y | A |
| 344 | Y-(C)-EENYLDPEQYQYFKYF-(K(acetyl))-Y | A |
| 345 | Y-(C)-EENYLDPEQYQYFIKF-(K(acetyl))-Y | A |
| 346 | Y-(C)-EENYLDPEQYQYFIYF-(K(acetyl))-K | A |
| 347 | M-(C)-EENYLDPEQYQYFIYF-(K(acetyl))-Y | A |
| 348 | Y-(C)-MENYLDPEQYQYFIYF-(K(acetyl))-Y | A |
| 349 | Y-(C)-EMNYLDPEQYQYFIYF-(K(acetyl))-Y | A |
| 350 | Y-(C)-EENMLDPEQYQYFIYF-(K(acetyl))-Y | A |
| 351 | Y-(C)-EENYMDPEQYQYFIYF-(K(acetyl))-Y | A |
| 352 | Y-(C)-EENYLMPEQYQYFIYF-(K(acetyl))-Y | A |
| 353 | Y-(C)-EENYLDMEQYQYFIYF-(K(acetyl))-Y | A |
| 354 | Y-(C)-EENYLDPMQYQYFIYF-(K(acetyl))-Y | A |
| 355 | Y-(C)-EENYLDPEQMQYFIYF-(K(acetyl))-Y | C |
| 356 | Y-(C)-EENYLDPEQYMYFIYF-(K(acetyl))-Y | A |
| 357 | Y-(C)-EENYLDPEQYQMFIYF-(K(acetyl))-Y | C |
| 358 | Y-(C)-EENYLDPEQYQYFMYF-(K(acetyl))-Y | A |
| 359 | Y-(C)-EENYLDPEQYQYFΠVIF-(K(acetyl))-Y | A |
| 360 | Y-(C)-EENYLDPEQYQYFIYF-(K(acetyl))-M | A |
| 361 | Q-(C)-YYLYWYLY-(K(acetyl))-I | C |
| 362 | L-(C)-YLYQYLYTT-(K(acetyl))-Y | C |
| 363 | L-(C)-YLYQYIYTA-(K(acetyl))-F | C |
| 364 | Q-(C)-LQYLYQYLF-(K(acetyl))-I | C |
| 365 | L-(C)-YLYQYLYQA-(K(acetyl))-F | C |
| 366 | D-(C)-QQYYLYLYLW-(K(acetyl))-I | C |
| 367 | H-(C)-QLEYLYTYVL-(K(acetyl))-I | C |
| 368 | Q-(C)-LIQDLYAYIF-(K(acetyl))-L | C |
| 369 | D-(C)-LQFLYLYLYQ-(K(acetyl))-T | C |
| 370 | D-(C)-QLYYLYQYVLT-(K(acetyl))-V | C |
| 371 | D-(C)-QLWYLYQYLIV-(K(acetyl))-A | C |
| 372 | Q-(C)-EQLKYLYQYVL-(K(acetyl))-L | C |
| 373 | Q-(C)-YNYQYIYQYIY-(K(acetyl))-I | C |
| 374 | Q-(C)-LQDLYRYLYLL-(K(acetyl))-A | C |
| 375 | D-(C)-LQFIYQYLYTV-(K(acetyl))-I | C |
| 376 | Q-(C)-IQDLYLYLYFL-(K(acetyl))-T | C |
| 377 | H-(C)-LHYLYQYLYTV-(K(acetyl))-Y | C |
| 378 | Q-(C)-PRQLYYLYLYLS-(K(acetyl))-I | C |
| 379 | N-(C)-QLYYTYLYLHTL-(K(acetyl))-F | C |
| 380 | Y-(C)-PPQLVYLYQYLY-(K(acetyl))-L | C |
| 381 | V-(C)-QEYYLYQYVYEL-(K(acetyl))-Y | C |
| 382 | P-(C)-DRQLYYLYQYIH-(K(acetyl))-T | C |
| 383 | Q-(C)-QLYYLYQYAYAL-(K(acetyl))-V | C |
| 384 | Q-(C)-QLYYLYQYTIAI-(K(acetyl))-T | C |
| 385 | Q-(C)-AYYHLYQYLHQL-(K(acetyl))-V | B |
| 386 | R-(C)-HHNFHHARQWGFFHHY-(K(acetyl))-R | B |
| 387 | R-(C)-HHNFHHARQHRFFHHY-(K(acetyl))-R | C |
| 388 | R-(C)-HHNFHHARQHGFFYHY-(K(acetyl))-R | C |
| 389 | R-(C)-HHNFHHARQHGFFWHY-(K(acetyl))-R | C |
| 390 | Y-(C)-EENYLDPEQYQYFHYF-(K(acetyl))-R | C |
| 391 | Y-(C)-EENYLDPEQYQYFHYF-(K(acetyl))-H | A |
| 392 | Y-(C)-HENYLDPEQYQYFHYF-(K(acetyl))-Y | A |
| 393 | Y-(C)-HENYLDPEQHQYFHYF-(K(acetyl))-Y | C |
| 394 | Y-(C)-HENYLDPEQYQYFHYF-(K(acetyl))-R | B |
| 395 | Y-(C)-HENYLDPEQYQYFHYF-(K(acetyl))-H | A |
| 396 | Y-(C)-EENYLDPEQHQYFIYF-(K(acetyl))-R | B |
| 397 | Y-(C)-EENYLDPEQHQYFIYF-(K(acetyl))-H | A |
| 398 | Y-(C)-HENYLDPEQHQYFIYF-(K(acetyl))-R | B |
| 399 | Q-(K(acetyl))-PQNFINPVQWRYFQHY-(C)-N | C |
| 400 | V-(C)-QQDNYFYPQQWFFFVHY-(K(acetyl))-H | A |
| 401 | Q-(C)-QFLNYVNHVQWHYFWVF-(K(acetyl))-S | C |
| 402 | P-(C)-EVYNYWYPTQWFFFTQF-(K(acetyl))-K | A |
| 403 | A-(C)-RRANWIHPIQWFFFHVY-(K(acetyl))-H | B |
| 404 | D-(C)-IRPYDQLQLIYQYLYQL-(K(acetyl))-K | C |
| 405 | Q-(C)-LTQRVYLRQLAYYRLYR-(K(acetyl))-I | C |
| 406 | P-(C)-EQQNFHSVIQWHFFAQY-(K(acetyl))-N | A |
| 407 | L-(C)-VQGNYQNPTQWYYFQLY-(K(acetyl))-W | B |
| 408 | H-(C)-SQHQTTYYQIYAYLHAI-(K(acetyl))-I | C |
| 409 | L-(C)-NNWENYFDWLQWSYYIAY-(K(acetyl))-E | A |
| 410 | W-(C)-QESSNYLAPLQWYFFNWY-(K(acetyl))-T | A |
| 411 | T-(C)-VDKDNWYNHVQWQYFFLY-(K(acetyl))-Q | A |
| 412 | Q-(C)-EYALQYYRFYVAQRHWVD-(K(acetyl))-L | C |
| 413 | Q-(C)-DQDAIQWVYDLQYYRVWH-(K(acetyl))-V | C |
| 414 | Y-(C)-NYEWDGARRQIAYYRIFL-(K(acetyl))-L | C |
| 415 | Y-(C)-DWVEVANARQLAYY⁻fLYR-(K(acetyl))-K | C |
| 416 | L-(C)-TQFPASQRYYFALYRLWH-(K(acetyl))-S | C |
| 417 | L-(C)-QIYLYLTALQQGFEDFYP-(K(acetyl))-N | C |
| 418 | D-(C)-LRQQVYQYLYILFQNDIA-(K(acetyl))-D | C |
| 419 | D-(C)-HTREDWQIHHLYQLLYQL-(K(acetyl))-Y | C |
| 420 | Y-(C)-EAQPYYFQLYRLWHRQE-(K(acetyl))-P | C |
| 421 | V-(C)-LDEQQALFHQYYRITRR-(K(acetyl))-V | C |
| 422 | Y-(C)-TQEQAFRQLAYYQIFRRS-(K(acetyl))-S | C |
| 423 | W-(C)-FTEGIQSTVQLTYSYLY-(K(acetyl))-L | C |
| 424 | V-(C)-TQPQQDFELIYQYLYQLA-(K(acetyl))-Q | C |
| 425 | G-(C)-GYTQLHYQHLFAWLHAR-(K(acetyl))-V | C |
| 426 | P-(C)-DPLNLQQWYTAVYQLWH-(K(acetyl))-R | C |
| 427 | E-(C)-DTADYPQEWIQDVYQYLY-(K(acetyl))-R | C |
| 428 | E-(C)-DQAQQVYALIYRYLYQLQ-(K(acetyl))-V | C |
| 429 | Y-(C)-HENYLDPEQYQYFHYF-(K(acetyl)) | C |
| 430 | (C)-HENYLDPEQYQYFHYF-(K(acetyl))-Y | A |
| 431 | (C)-HENYLDPEQYQYFHYF-(K(acetyl)) | C |
| 432 | (C)-ENYLDPEQYQYFHYF-(K(acetyl))-Y | C |

### <Example 3: Evaluation of binding affinity of cyclic peptide (chemically synthesized product)>

The binding affinity of the cyclic peptide to FGFR was evaluated using a surface plasmon resonance (SPR) method.

The cyclic peptide used for the evaluation was custom-synthesized at Toray Research Center, Inc.

The binding affinity of the chemically synthesized cyclic peptide to FGFR was evaluated by the following procedure.

A CM5 sensor chip (Cytiva, 29149603) was set in Biacore-T200 (manufactured by Cytiva) which is a surface plasmon resonance device, and an HBS-EP buffer (Cytiva, BR100826) was added as a running buffer at a flow rate of 10 µL/min to equilibrate the flow channel. Next, 70 µL of a mixed aqueous solution of 0.2 mol/L 1-(3-dimethylaminopropyl)-3-ethylcarbodiimide (EDC) and 0.05 mol/L N-hydroxysuccinimide (NHS) was added to activate the sensor chip. Then, Recombinant Human FGFR2 alpha (IIIc) Fc Chimera Protein CF (R&D systems, 712-FR-050) prepared at 50 nmol/L in an Acetate 4.5 buffer (Cytiva, BR100350) was added thereto to perform immobilization at about 5000 RU. Thereafter, the blocking treatment was performed with an ethanolamine solution. In addition, in another flow channel of the same sensor chip, blocking treatment was performed immediately after activation without immobilizing FGFR.

A cyclic peptide diluted to 10 nmol/L using HBS-EP was added to each flow channel of the FGFR-immobilized sensor chip produced above at 25°C for 10 minutes, then HBS-EP was allowed to flow as a running buffer for 30 minutes, and the binding of the cyclic peptide to FGFR was measured. Thereafter, a regeneration treatment of removing the bound cyclic peptide was performed by repeating the flow of a regeneration liquid in which sodium chloride powder was dissolved in Glysine 1.5 (Cytiva, BR100354) to a final concentration of 0.15 mol/L for 1 minute in each flow channel twice. The binding amount of the cyclic peptide was evaluated from the difference between the measured value of Biacore-T200 in the flow channel in which FGFR was immobilized and the measured value of Biacore-T200 in the flow channel in which FGFR was not immobilated, in a case where a 10 nmol/L cyclic peptide was allowed to flow.

The binding amount of the cyclic peptide evaluated above was normalized to the binding amount of the cyclic peptide molecules per one molecule of FGFR using the molecular weights of the cyclic peptide and FGFR, and the binding affinity to FGFR was evaluated according to the following evaluation criteria. The evaluation result preferably satisfies the evaluation criteria A, B, or C.

### (Evaluation criteria for FGFR binding affinity)

A: The binding amount is 0.7 molecules of the cyclic peptide/one molecule of FGFR or more.
B: The binding amount is 0.5 molecules of the cyclic peptide/one molecule of FGFR or more and less than 0.7 molecules of cyclic peptide/one molecule of FGFR.
C: The binding amount is 0.1 molecules of cyclic peptide/one molecule of FGFR or more and less than 0.5 molecules of cyclic peptide/one molecule of FGFR.
D: The binding amount is less than 0.1 molecules of the cyclic peptide/one molecule of FGFR.

Table 5 shows the evaluation results.

**[Table 5]**

| SEQ ID NO: | Sequence | Evaluation result of binding affinity to FGFR |
|---|---|---|
| 433 | Q-(Hcy)-PQNFINPVQWRYFQHY-(K(acetyl))-NKKK | A |
| 434 | Q-(Hcy)-PQNFINPVQWRYFQHY-(Orn(acetyl))-NKKK | A |
| 435 | Q-(Hcy)-PQNFINPVQWRYFQHY-(Dab(acetyl))-NKKK | A |
| 437 | Q-(K(acetyl))-PQNFINPVQWRYFQHY-(Hcy)-NKKK | A |
| 438 | Q-(K(acetyl))-PQNFINPVQWRYFQHY-(Hcy)-NKKK | A |
| 439 | Q-(Orn(acetyl))-PQNFINPVQWRYFQHY-(Hcy)-NKKK | A |
| 440 | Q-(Dab(acetyl))-PQNFINPVQWRYFQHY(Hcy)-NKKK | A |
| 441 | Q-(Dap(acetyl))-PQNFINPVQWRYFQHY-(Hcy)-NKKK | A |
| 442 | Y-(Hcy)-EENYLDPEQYQYFIYF-(K(acetyl))-YKKK | A |
| 443 | Y-(Hcy)-EENYLDPEQYQYFIYF-(Orn(acetyl))-YKKK | A |
| 444 | Y-(Hcy)-EENYLDPEQYQYFIYF-(Dab(acetyl))-YKKK | B |
| 446 | Y-(K(acetyl))-EENYLDPEQYQYFIYF-(Hcy)-YKKK | A |
| 447 | Y-(Orn(acetyl))-EENYLDPEQYQYFIYF-(Hcy)-YKKK | A |
| 448 | Y-(Dab(acetyl))-EENYLDPEQYQYFIYF-(Hcy)-YKKK | A |
| 449 | Y-(Dap(acetyl))-EENYLDPEQYQYFIYF-(Hcy)-YKKK | A |
| 450 | Y-(C)-EENYLDPEQYQYFIYF-(K(acetyl))-YKKK | A |
| 451 | Y-(C)-EENYLDPEQYQYFIYF-(C)-YKKK | D |
| 452 | Y-(Hcy)-HENYLDPEQYQYFHYF-(K(acetyl))-YKKK | A |
| 453 | Y-(Hcy)-HENYLDPEQYQYFHYF-(K(acetyl))-KKK | C |
| 454 | (Hcy)-HENYLDPEQYQYFHYF-(K(acetyl))-YKKK | C |

### <Example 4: Evaluation of FGFR versatility of cyclic peptide (chemically synthesized product)>

The binding of the cyclic peptide to various types of FGFRs was evaluated using a surface plasmon resonance (SPR) method.

The cyclic peptide used for the evaluation was custom-synthesized at Toray Research Center, Inc.

The binding affinity of the chemically synthesized cyclic peptide to FGFR was evaluated by the following procedure.

A CM5 sensor chip (Cytiva, 29149603) was set in Biacore-T200 (manufactured by Cytiva) which is a surface plasmon resonance device, and an HBS-EP buffer (Cytiva, BR100826) was added as a running buffer at a flow rate of 10 µL/min to equilibrate the flow channel. Next, 70 µL of a mixed aqueous solution of 0.2 mol/L 1-(3-dimethylaminopropyl)-3-ethylcarbodiimide (EDC) and 0.05 mol/L N-hydroxysuccinimide (NHS) was added to activate the sensor chip. Then, a cyclic peptide prepared to 2 mg/mL in HBS-EP buffer was added thereto to perform immobilization at about 200 RU. Thereafter, the blocking treatment was performed with an ethanolamine solution. In addition, in another flow channel of the same sensor chip, blocking treatment was performed immediately after activation without immobilizing FGFR.

After various FGFRs (FGFR1 Protein, Human, Recombinant (His Tag) (Sino Biological, 10616-H08H), FGFR2 Protein, Human, Recombinant (His Tag) (Sino Biological, 10824-H08H), FGFR3 Protein, Human, Recombinant (ECD, His Tag) (Sino Biological, 16044-H08H), FGFR4 Protein, Human, Recombinant (His Tag) (Sino Biological, 10538-H08H)) diluted with HBS-EP to 1 µmol/L at 25°C was added to each flow channel of the cyclic peptide-immobilized sensor chip prepared above, for 10 minutes, HBS-EP was allowed to flow as a running buffer for 30 minutes, and the binding of various FGFRs to the cyclic peptide was measured. Thereafter, a regeneration treatment of removing the bound FGFR was performed by repeating the flow of a regeneration liquid in which sodium chloride (final concentration: 0.4 mol/L) and a sodium dodecyl sulfate aqueous solution (mixed concentration: 1%, final concentration: 0.2%) were dissolved in Glysine 1.5 (Cytiva, BR100354) for 1 minute in each flow channel twice. The binding amount of the cyclic peptide was evaluated from the difference between the measured value of Biacore-T200 in the flow channel in which the cyclic peptide was immobilized and the measured value of Biacore-T200 in the flow channel in which the cyclic peptide was not immobilized, in a case where 1 µmol/L FGFR was allowed to flow.

The binding amount of FGFR evaluated above was normalized to the binding amount of FGFR molecules per one molecule of the cyclic peptide using the molecular weights of the cyclic peptide and FGFR, and the binding affinity to FGFR was evaluated according to the following evaluation criteria. The evaluation result preferably satisfies the evaluation criteria A, B, or C.

### (Evaluation criteria for FGFR binding affinity)

A: The binding amount is 0.05 molecules of FGFR/one molecule of the cyclic peptide or more.
B: The binding amount is 0.01 molecules of FGFR/one molecule of the cyclic peptide or more and less than 0.05 molecules of FGFR/one molecule of the cyclic peptide.
C: The binding amount is 0.005 molecules of FGFR/one molecule of the cyclic peptide or more and less than 0.01 molecules of FGFR/one molecule of the cyclic peptide.
D: The binding amount is less than 0.005 molecules of FGFR/one molecule of the cyclic peptide.

The evaluation results are shown in Table 6.

**[Table 6]**

| SEQ ID NO: | Sequence | Evaluation result of binding affinity to FGFR1 | Evaluation result of binding affinity to FGFR2 | Evaluation result of binding affinity to FGFR3 | Evaluation result of binding affinity to FGFR4 |
|---|---|---|---|---|---|
| 450 | Y-(C)- EENYLDPEQYQYFIYF- (K(acetyl))-YKKK | B | A | B | B |

From this result, it was shown that the cyclic peptide according to the embodiment of the present invention can be bound to various types of FGFRs.

### <Example 5: Evaluation of stability of cyclic peptide>

The molecular stability of the cyclic peptide was evaluated by analyzing an aqueous solution of the cyclic peptide treated with a reducing agent by liquid chromatography (LC).

The reducing agent treatment was performed under the following conditions based on the concentration of glutathione contained in the cells. 25 µL of a 0.2 mg/mL cyclic peptide aqueous solution was prepared, 58.8 µL of a 12.6 mmol/L DTT aqueous solution was added to the aqueous solution, and the mixture was incubated at room temperature for 1 hour to obtain an aqueous solution of the cyclic peptide treated with a reducing agent. The DTT aqueous solution was used by diluting a 1 mol/L (+-)-dithiothreitol (DTT) solution (FUJIFILM Wako Pure Chemical Corporation, 044-33871) with water. The total area of all the peaks in LC/MS of the cyclic peptide before the reducing agent treatment was set to 100%, and the proportion of the total area of all the peaks in LC/MS of the aqueous solution of the cyclic peptide treated with the reducing agent was determined to calculate the residual rate of the cyclic peptide, and the molecular stability was evaluated according to the following evaluation criteria. The evaluation result preferably satisfies the evaluation criteria A, B, or C.

### (Evaluation criteria for residual rate of cyclic peptide)

A: The residual rate of cyclic peptide is 90% or more.
B: The residual rate of the cyclic peptide is 70% or more and less than 90%
C: The residual rate of the cyclic peptide is 50% or more and less than 70%
D: The residual rate of the cyclic peptide is less than 50%

The LC used for the evaluation of molecular stability was set to the following conditions.
· LC device: Prominence series (pump, column oven, autosampler, detector) (manufactured by Shimadzu Corporation)
· Detector: photodiode array detector (SPD-M20A), measurement wavelength of 280 nm
· Column: TSKgel ODS-100V, inner diameter of 4.6 mm × length of 150 mm, particle diameter of 5 µm (manufactured by Tosoh Corporation)
· Eluent A: solution containing 0.1% trifluoroacetic acid as solute, and solvent is 100% water
· Eluent B: solution containing 0.1% trifluoroacetic acid as solute, and solvent is 100% acetonitrile
· Flow rate: 1.0 mL/min
· Injection amount: 25 µL
· Gradient: 20% to 50%: Eluent B (0 to 15 minutes), 100%: Eluent B (15 minutes)
· Column temperature: 40°C

The evaluation results are shown in Table 7. The amino acid residues described in parentheses indicate amino acid residues constituting a cyclized structure by a thioether bond or a disulfide bond between side chains. For example, K(acetyl) refers to a structure in which a lysine residue side chain is modified with a haloacetyl group, and the cyclic peptide has a cyclized structure in which a halogen atom of the haloacetyl group is substituted with a thiol group of another residue.

**[Table 7]**

| SEQ ID NO: | Sequence | Evaluation result of reductive stability |
|---|---|---|
| 433 | Q-(Hcy)-PQNFINPVQWRYFQHY-(K(acetyl))-NKKK | A |
| 434 | Q-(Hcy)-PQNFINPVQWRYFQHY-(Orn(acetyl))-NKKK | A |
| 435 | Q-(Hey)-PQNFINPVQWRYFQHY-(Dab(acetyl))-NKKK | A |
| 437 | Q-(K(acetyl))-PQNFINPVQWRYFQHY-(Hcy)-NKKK | A |
| 438 | Q-(K(acetyl))-PQNFINPVQWRYFQHY-(Hcy)-NKKK | A |
| 439 | Q-(Orn(acetyl))-PQNFINPVQWRYFQHY(Hcy)-NKKK | A |
| 440 | Q-(Dab(acetyl))-PQNFINPVQWRYFQHY-(Hcy)-NKKK | A |
| 441 | Q-(Dap(acetyl))-PQNFINPVQWRYFQHY-(Hcy)-NKKK | A |
| 442 | Y-(Hcy)-EENYLDPEQYQYFIYF-(K(acetyl))-YKKK | A |
| 443 | Y (Hcy)-EENYLDPEQYQYFIYF-(Orn(acetyl))-YKKK | A |
| 444 | Y-(Hcy)-EENYLDPEQYQYFIYF-(Dab(acetyl))-YKKK | A |
| 446 | Y-(K(acetyl))-EENYLDPEQYQYFIYF-(Hcy)-YKKK | A |
| 447 | Y-(Orn(acetyl))-EENYLDPEQYQYFIYF-(Hcy)-YKKK | A |
| 448 | Y-(Dab(acetyl))-EENYLDPEQYQYFIYF-(Hcy)-YKKK | A |
| 449 | Y-(Dap(acetyl))-EENYLDPEQYQYFIYF-(Hcy)-YKKK | A |
| 450 | Y-(C)-EENYLDPEQYQYFIYF-(K(acetyl))-YKKK | B |
| 451 | Y-(C)-EENYLDPEQYQYFIYF-(C)-YKKK | D |
| 452 | Y-(Hcy)-HENYLDPEQYQYFHYF-(K(acetyl))-YKKK | A |
| 453 | Y-(Hcy)-HENYLDPEQYQYFHYF-(K(acetyl))-KKK | A |
| 454 | (Hcy)-HENYLDPEQYQYFHYF-(K(acetyl))-YKKK | B |

From this result, it was shown that the cyclic peptide cyclized by a thioether bond is more stable than the cyclic peptide cyclized by a disulfide bond.

In addition, the stability of the cyclic peptide (SEQ ID NO: 450) containing a cysteine residue in the cyclized portion and the cyclic peptide (SEQ ID NO: 442) containing a homocysteine residue in the cyclized portion was compared under the following alkali treatment, which is a more severe condition.

The alkali treatment was performed by the following method. 25 µL of a 0.2 mg/mL cyclic peptide aqueous solution was prepared, 58.8 µL of a 0.5 mol/L sodium hydroxide aqueous solution was added to the aqueous solution, and the mixture was incubated at room temperature for 3 hours to obtain an aqueous solution of the cyclic peptide alkali-treated. The total area of all the peaks in LC/MS of the cyclic peptide before the alkali treatment was set to 100%, and the proportion of the total area of all the peaks in LC/MS of the aqueous solution of the cyclic peptide alkali-treated was determined to calculate the residual rate of the cyclic peptide. It was found that the residual rate of SEQ ID NO: 450 was 36%, the residual rate of SEQ ID NO: 442 was 61%, and the cyclic peptide containing a homocysteine residue in the cyclized portion was more stable than the cyclic peptide containing a cysteine residue in the cyclized portion.

The LC conditions used for the evaluation of molecular stability are the same as those in the measurement of the above-described cyclic peptide treated with a reducing agent.

### <Example 6: Evaluation of cell proliferation-promoting effect of cyclic peptide complex>

The FGFR phosphorylation ability of the cyclic peptide complex was evaluated using the BaF3 cell proliferation-promoting effect in which FGFR was stably expressed.

BaF3 cells stably expressing FGFR were established by introducing a vector (human: Sino Biologicals, HG10824-UT, bovine: sequence of Accession No. F1MNW2 was introduced into pCDNA3.1/Hygro(+) Mammalian Expression Vector (Thermo, V87020), porcine: sequence of Accession No. A0A4X1SNN3 was introduced into pCDNA3.1/Hygro(+) Mammalian Expression Vector (Thermo, V87020), and avian: sequence of Accession No. A0A8V0XB17 was introduced into pCDNA3.1/Hygro(+) Mammalian Expression Vector (Thermo, V87020)) into which FGFR gene had been incorporated, into BaF3 cells using NucleoFector (Lonza) and performing drug selection of cells into which the FGFR gene had been introduced using hygromycin.

The cyclic peptide complex used for the evaluation was custom-synthesized at Toray Research Center, Inc.

The evaluation of the proliferation-promoting effect of BaF3 cells in which FGFR is stably expressed was performed by the following method. First, RPMI-1640 with L-Glutamine and Phenol Red (FUJIFILM Wako Pure Chemical Corporation, 189-02025) and Fetal Bovine Serum (Thermo, 10270-106) were mixed at a volume ratio of 9:1, and then heparin (STEM CELL, 07980) was added thereto such that the final concentration thereof was 8 µg/mL, thereby preparing a culture medium for evaluation (hereinafter, described as RPMI (10% FBS) medium). Next, the BaF3 cells in which FGFR had been stably expressed were washed twice with RPMI (10% FBS) culture medium, suspended in RPMI (10% FBS) culture medium in which the cyclic peptide complex had been dissolved to a final concentration of 0.001 to 10 nmol/L, and seeded in a 384-well plate. In this case, a condition in which the cyclic peptide complex was not added as a negative control was prepared. After culturing at 37°C (5% CO₂) for 2 nights, Hoechst 33342, Trihydrochloride, Trihydrate - 10 mg/mL Solution in Water (Thermo, H3570), -Cellstain (registered trademark)- Calcein-AM solution (1 mg/mL DMSO solution) (DOJINDO, C396), and -Cellstain (registered trademark)- PI solution (DOJINDO, P378) were each added to RPMI (10% FBS) culture medium in a volume amount of 1/1,000 at the final concentration, and live/dead staining of the cells were performed by allowing to stand at room temperature for 1 hour, and imaging was performed using a confocal microscope (Yokogawa Electric Corporation, CQ1). The number of viable cells was calculated from the imaging data, and the cell proliferation-promoting effect was evaluated according to the following evaluation criteria. The number of viable cells in the negative control was about 100 cells/well. The evaluation result preferably satisfies the evaluation criteria A, B, or C.

### (Evaluation criteria for cell proliferation-promoting effect)

At an addition concentration of the cyclic peptide complex having the largest number of viable cells, evaluation criteria is as follows.
A: The number of viable cells is 1,000 cells/well or more.
B: The number of viable cells is 400 cells/well or more and less than 1,000 cells/well.
C: The number of viable cells is 200 cells/well or more and less than 400 cells/well.
D: The number of viable cells is less than 200 cells/well.

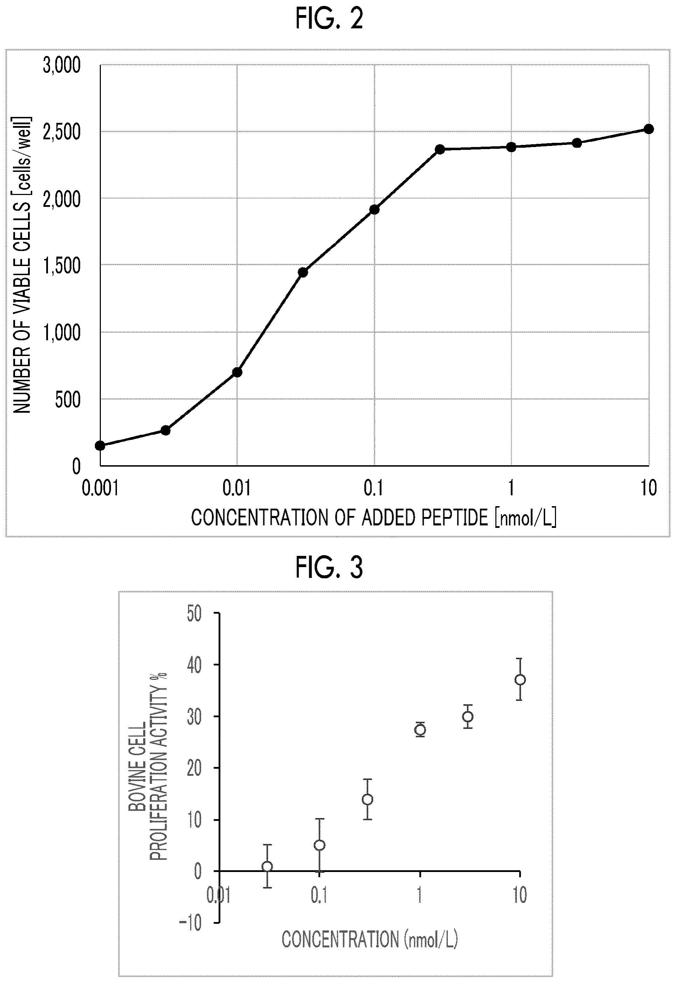

The evaluation results are shown in Table 8 and Fig. 2. The amino acid residues described in parentheses indicate amino acid residues constituting a cyclized structure by a thioether bond or a disulfide bond between side chains. For example, K(acetyl) refers to a structure in which a lysine residue side chain is modified with a haloacetyl group, and the cyclic peptide has a cyclized structure in which a halogen atom of the haloacetyl group is substituted with a thiol group of another residue.

**[Table 8]**

| SEQ ID NO: | Sequence | Evaluation result of cell proliferative properties | | | | Modification information |
|---|---|---|---|---|---|---|
| | | Human | Bovine | Porcine | Avian | |
| 455 | K-(C₁)- PQNFINPVQWRYF QHY-(K(acetyl)₁)- NGSGSGSSGSSSGS SK-(C₂)- PQNFINPVQWRYF QHY-(K(acetyl)₂)-N | A | B | A | C | Ring formation between C₁-K(acetyl)₁ and ring formation between C₂-K(acetyl)₂ |
| 456 | Q-(C)- PQNFINPVQWRYF QHY-(K(acetyl))-NK | A | A | A | A | N-terminal acetylation, crosslinking between side chain amino groups of K via Bis-NHS-PEG3 |
| 457 | Q-(C)- PQNFINPVQWRYF QHY-(K(acetyl))-NK | A | A | A | A | N-terminal acetylation, crosslinking between side chain amino groups of K via Bis-NHS-PEG6 |
| 458 | Q-(C)- PQNFINPVQWRYF QHY-(K(acetyl))-NK | A | A | A | A | N-terminal acetylation, crosslinking between side chain amino groups of K via Bis-NHS-PEG9 |
| 459 | Q-(C)- PQNFINPVQWRYF QHY-(K(acetyl))- N/Ac-Q-(C)- PQNFINPVQWRYF QHY-(K(acetyl))-NK | A | B | A | B | N-terminal acetylation of second ring unit, crosslinking between N-terminal amino group of first ring unit and side chain amino group of K of second ring unit via Bis-NHS-PEG11 |
| 460 | Y-(C)- EENYLDPEQYQYF IYF-(K(acetyl))-YK | A | A | A | A | N-terminal acetylation, dimerization by crosslinking between side chain amino groups of K via Bis-NHS-PEG9 |
| 461 | Y-(Hcy)- HENYLDPEQYQYF HYF-(K(acetyl))-YK | B | B | B | B | N-terminal acetylation, C-terminal amidation, crosslinking between side chain amino groups of K via Bis-NHS-PEG7 |
| 462 | Y-(Hcy)- HENYLDPEQYQYF HYF-(K(acetyl))-YK | B | B | B | C | N-terminal acetylation, C-terminal amidation, crosslinking between side chain amino groups of K via Bis-NHS-PEG9 |
| 463 | Y-(Hcy)- HENYLDPEQYQYF HYF-(K(acetyl))-YK | B | B | B | B | N-terminal acetylation, C-terminal amidation, crosslinking between side chain amino groups of K via Bis-NHS-PEG11 |

From this result, it was shown that the cyclic peptide complex to which the cyclic peptide according to the embodiment of the present invention was linked was capable of phosphorylating various FGFRs derived from animals.

The structures of the cyclic peptide complexes of SEQ ID NOs: 455 to 463 are shown below.

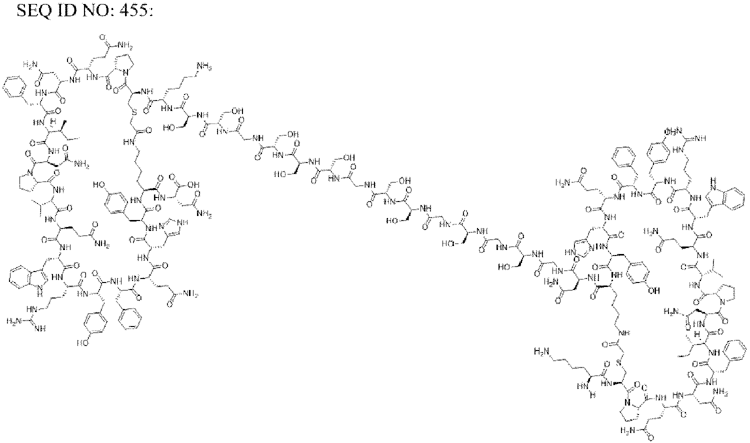

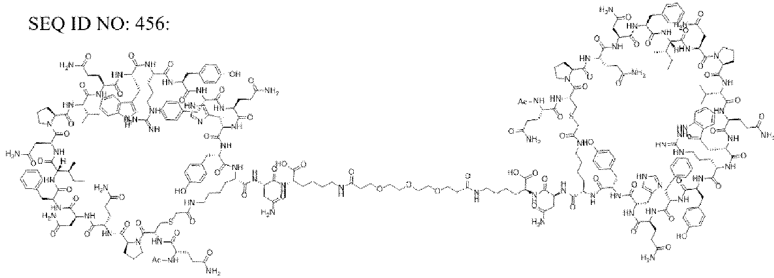

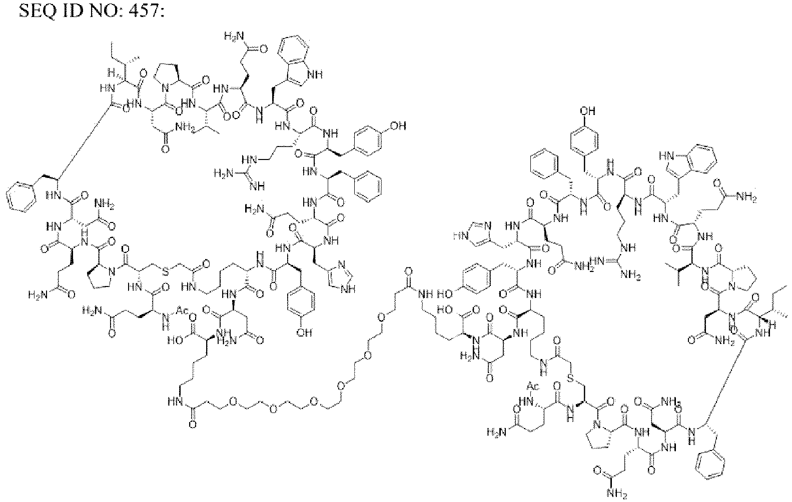

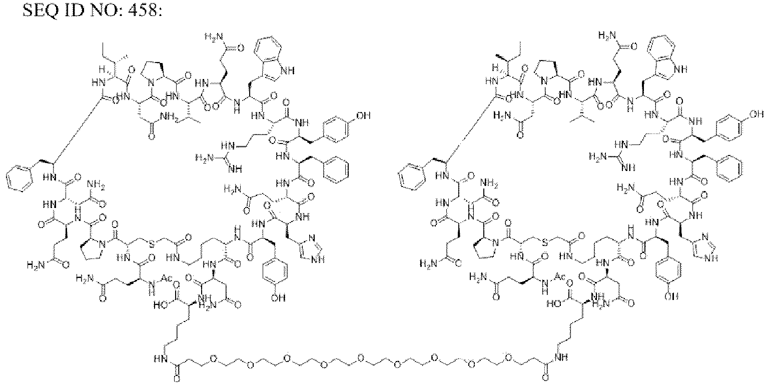

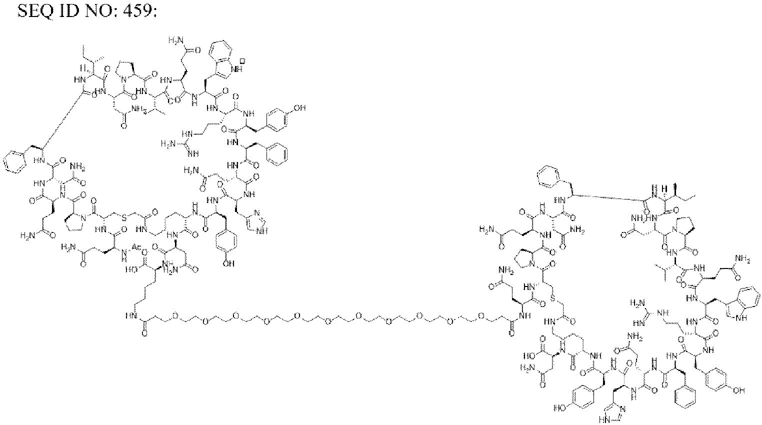

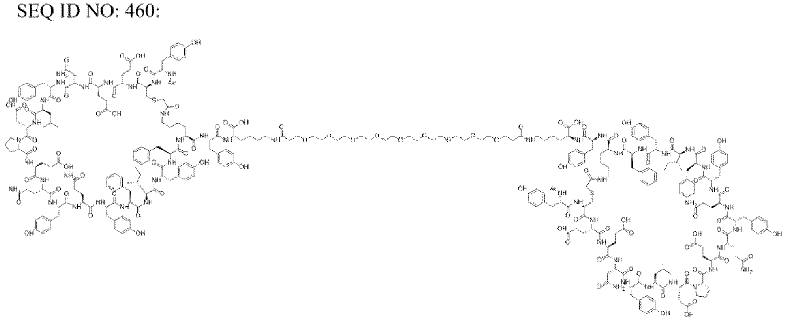

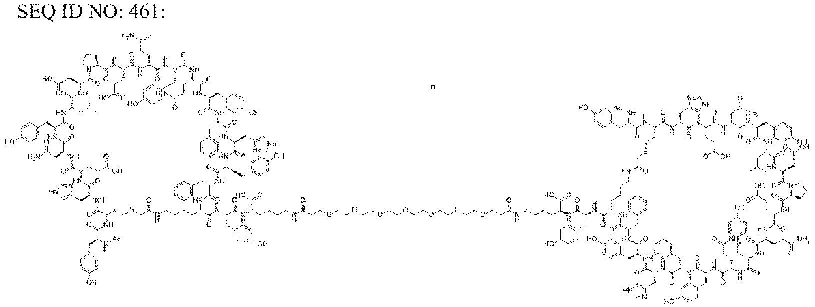

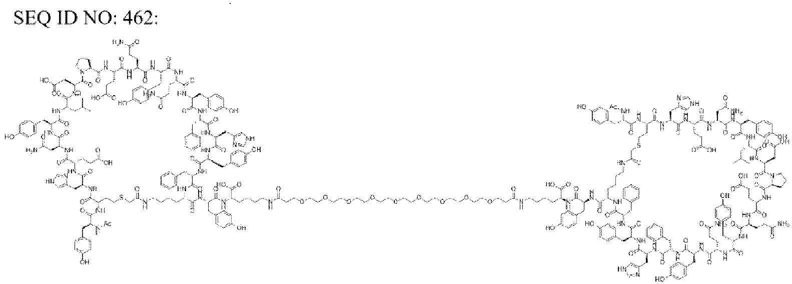

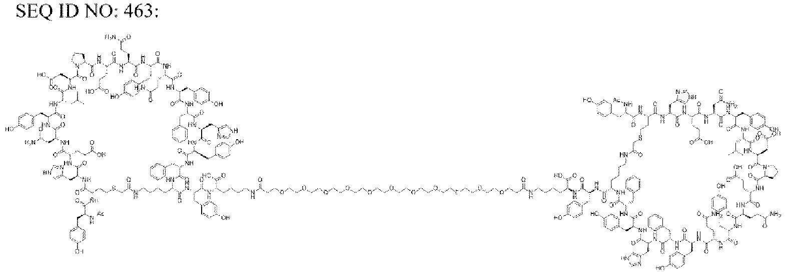

### <Example 7: Evaluation of bovine cell proliferation-promoting effect of cyclic peptide complex>

The bovine cells (myoblasts) proliferation-promoting effect of the cyclic peptide complex was evaluated.

Bovine myoblasts were separated from bovine muscle tissue by the following method. First, muscle tissue of Japanese Black cattle was finely chopped in HBSS(-) (phenol red-free) (FUJIFILM Wako Pure Chemical Corporation, 085-09355), and then pronase from Streptomyces griseus (Sigma-Aldrich, 10165921001) was added thereto to a final concentration of 1 mg/mL, and the mixture was allowed to stand at 37°C for 1 hour. After allowing to stand, HBSS containing 10% FBS was added thereto, and the cells were acquired by centrifugation. The acquired cells were suspended in D-MEM (high glucose) (containing L-glutamine and phenol red) (FUJIFILM Wako Pure Chemical Corporation, 044-29765) containing 10% final concentration of bovine serum and 30 ng/mL bFGF. Suspended cells were cultured in a culture flask coated with an iMatrix-511 solution (FUJIFILM Wako Pure Chemical Corporation, 381-07363) at 0.55 µg/cm². Bovine myoblasts that had been subcultured two or more times after the start of the culture were used for the evaluation.

The evaluation of the proliferation-promoting effect on bovine myoblasts was performed by the following method. The subcultured bovine myoblasts were peeled using trypsin-EDTA (0.05%) and phenol red (Thermo, 25300-054), suspended in 10% FBS-containing D-MEM (high glucose) (containing L-glutamine and phenol red) diluted to a final concentration of 30 ng/mL of bFGF or 0.03 to 10 nmol/L of the cyclic peptide complex, and seeded on a 96-well plate coated with iMatrix-511 at 0.55 µg/cm². In this case, conditions in which bFGF and the cyclic peptide complex were not added as a negative control were prepared. After culturing at 37°C (5% CO₂) for 4 days, the supernatant of the culture medium was removed, and a 10% FBS-containing D-MEM (high glucose) (containing L-glutamine and phenol red) was added thereto, in which Hoechst 33342, Trihydrochloride, Trihydrate - 10 mg/mL Solution in Water (Thermo, H3570) and -Cellstain (registered trademark)- Calcein-AM solution (1 mg/mL DMSO solution) (DOJINDO, C396) were added in a volume amount of 1/10,000 and 1/4,000 at final concentration, and all cells and the viable cells were stained by allowing to stand at 37°C (5% CO₂) for 30 minutes, and imaged with a confocal microscope (Yokogawa Electric Corporation, CQ1). The number of viable cells was calculated from the imaging data and normalized by setting the number of viable cells in the well to which 30 ng/mL bFGF was added to 100, and setting the number of viable cells in the negative control well to 0. The evaluation results are shown in Fig. 3.

The cyclic peptide complex (SEQ ID NO: 458) exhibits a bovine myoblast proliferation action of about 40% of 30 ng/mL bFGF, which indicates that the cyclic peptide complex according to the embodiment of the present invention can also be used for culturing bovine myoblasts.

[Sequence list] International application 22F01536W1JP24010164_41.xml based on International Patent Cooperation Treaty

## Claims

1. A cyclic peptide or a salt thereof, the cyclic peptide being a peptide having an amino acid sequence represented by X1-Xm-X2-Xn-X3,
wherein the cyclic peptide contains a cyclized portion that is cyclized via covalent bonding, and
the cyclized portion includes a structure represented by Formula (2), 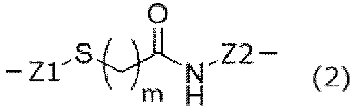
X1 represents a glutamine residue or a histidine residue,
Xm represents a peptide residue containing any 2 to 4 amino acid residues,
X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
Xn represents an amino acid residue or a peptide residue containing any 1 to 3 amino acid residues, and
X3 represents an amino acid residue containing an aromatic residue on a side chain,
in Formula (2),
Z1 and Z2 each independently represent a linking group, and
m represents an integer of 1 to 10.

2. The cyclic peptide or a salt thereof according to claim 1,
wherein the cyclic peptide is represented by Formula (1-1) or Formula (1-2), 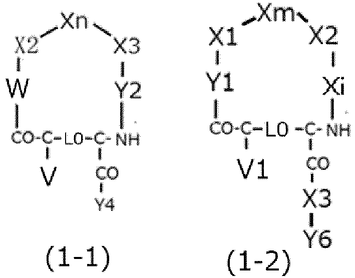
in the formulae,
W represents -Xm-X1-Y1- or Xk, where Xm is bonded to X2 and Y1 is bonded to CO,
in a case where W represents -Xm-X1-Y1-, V represents -NH-Y3 and L0 represents L, or V represents R and L0 represents L1,
in a case where W represents Xk, V represents -NH-X1-Y5 and L0 represents L,
V1 represents -NH-Y3 or R,
in a case where V1 represents -NH-Y3, L0 represents L, and in a case where V1 represents R, L0 represents L1,
X1 represents a glutamine residue or a histidine residue,
Xm represents a peptide residue containing any 2 to 4 amino acid residues,
Xk represents a peptide residue containing any 1 to 3 amino acid residues,
X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
Xn represents an amino acid residue or a peptide residue containing any 1 to 3 amino acid residues,
Xi represents a single bond, or an amino acid residue or a peptide residue containing any 1 or 2 amino acid residues,
X3 represents an amino acid residue containing an aromatic residue on a side chain,
Y1 represents a single bond, or an amino acid residue or a peptide residue containing any 1 to 10 amino acid residues,
Y2 represents a single bond, or an amino acid residue or a peptide residue containing any 1 to 12 amino acid residues,
Y3 represents a hydrogen atom, or an amino acid residue or a peptide residue containing any 1 to 7 amino acid residues,
Y4 represents OH, or an amino acid residue or a peptide residue containing any 1 to 7 amino acid residues,
Y5 represents a hydrogen atom, or an amino acid residue or a peptide residue containing any 1 to 7 amino acid residues,
Y6 represents OH, or an amino acid residue or a peptide residue containing any 1 to 7 amino acid residues,
L and L1 represent a cyclized portion,
R represents a side chain structure of an amino acid, and
an amino terminal of X1 is bonded to a carboxyl terminal of Y1, and a carboxyl terminal of X1 is bonded to an amino terminal of Xm.

3. The cyclic peptide or a salt thereof according to claim 1,
wherein the cyclic peptide is represented by Formula (1A), Formula (1B), or Formula (1C), 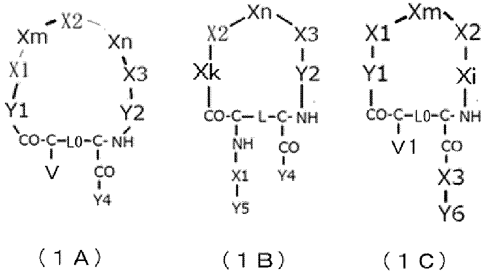
in the formulae,
X1 represents a glutamine residue or a histidine residue,
Xm represents a peptide residue containing any 2 to 4 amino acid residues,
Xk represents a peptide residue containing any 1 to 3 amino acid residues,
X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
Xn represents an amino acid residue or a peptide residue containing any 1 to 3 amino acid residues,
Xi represents a single bond, or an amino acid residue or a peptide residue containing any 1 or 2 amino acid residues,
X3 represents an amino acid residue containing an aromatic residue on a side chain,
Y1 represents a single bond, or an amino acid residue or a peptide residue containing any 1 to 10 amino acid residues,
Y2 represents a single bond, or an amino acid residue or a peptide residue containing any 1 to 12 amino acid residues,
Y3 represents a hydrogen atom, or an amino acid residue or a peptide residue containing any 1 to 7 amino acid residues,
Y4 represents OH, or an amino acid residue or a peptide residue containing any 1 to 7 amino acid residues,
Y5 represents a hydrogen atom, or an amino acid residue or a peptide residue containing any 1 to 7 amino acid residues,
Y6 represents OH, or an amino acid residue or a peptide residue containing any 1 to 7 amino acid residues,
V represents -NH-Y3 or R, in a case where V is -NH-Y3, L0 represents L, and in a case where V is R, L0 represents L1,
V1 represents -NH-Y3 or R, in a case where V1 is -NH-Y3, L0 represents L, and in a case where V1 is R, L0 represents L1,
L and L1 represent a cyclized portion,
R represents a side chain structure of an amino acid, and
an amino terminal of X1 is bonded to a carboxyl terminal of Y1, and a carboxyl terminal of X1 is bonded to an amino terminal of Xm.

4. The cyclic peptide or a salt thereof according to claim 1,
wherein the cyclic peptide is represented by Formula (1A1), Formula (1A2), Formula (1B), Formula (1C1), or Formula (1C2), 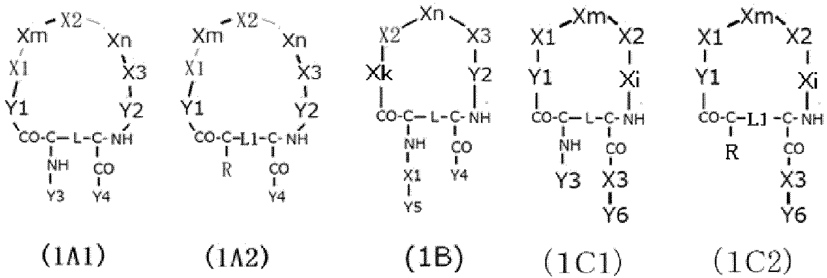
in the formulae,
X1 represents a glutamine residue or a histidine residue,
Xm represents a peptide residue containing any 2 to 4 amino acid residues,
Xk represents a peptide residue containing any 1 to 3 amino acid residues,
X2 represents an alanine residue, a leucine residue, an isoleucine residue, a phenylalanine residue, a tyrosine residue, a tryptophan residue, a glutamine residue, a serine residue, a threonine residue, an asparagine residue, a glutamic acid residue, an arginine residue, or a histidine residue,
Xn represents an amino acid residue or a peptide residue containing any 1 to 3 amino acid residues,
Xi represents a single bond, or an amino acid residue or a peptide residue containing any 1 or 2 amino acid residues,
X3 represents an amino acid residue containing an aromatic residue on a side chain,
Y1 represents a single bond, or an amino acid residue or a peptide residue containing any 1 to 10 amino acid residues,
Y2 represents a single bond, or an amino acid residue or a peptide residue containing any 1 to 12 amino acid residues,
Y3 represents a hydrogen atom, or an amino acid residue or a peptide residue containing any 1 to 7 amino acid residues,
Y4 represents OH, or an amino acid residue or a peptide residue containing any 1 to 7 amino acid residues,
Y5 represents a hydrogen atom, or an amino acid residue or a peptide residue containing any 1 to 7 amino acid residues,
Y6 represents OH, or an amino acid residue or a peptide residue containing any 1 to 7 amino acid residues,
L and L1 represent a cyclized portion,
R represents a side chain structure of an amino acid, and
an amino terminal of X1 is bonded to a carboxyl terminal of Y1, and a carboxyl terminal of X1 is bonded to an amino terminal of Xm.

5. The cyclic peptide or a salt thereof according to claim 1,
wherein Xm represents a group represented by -Xo-X4-,
Xo represents an amino acid residue or a peptide residue containing any 1 to 3 amino acid residues, and
X4 represents an alanine residue, an isoleucine residue, a phenylalanine residue, a tyrosine residue, an asparagine residue, a histidine residue, or a methionine residue.

6. The cyclic peptide or a salt thereof according to claim 1,
wherein Xm represents a group represented by -Xo-X4-,
Xo represents an amino acid residue or a peptide residue containing any 1 to 3 amino acid residues,
X4 represents an isoleucine residue, a phenylalanine residue, a tyrosine residue, an asparagine residue, a histidine residue, or a methionine residue,
Xk represents a group represented by -Xj-X4-, and
Xj represents a single bond, or an amino acid residue or a peptide residue containing any 1 or 2 amino acid residues.

7. The cyclic peptide or a salt thereof according to claim 1,
wherein Xm represents a group represented by -X5-X6-X4-,
X5 represents an alanine residue, a proline residue, a leucine residue, an isoleucine residue, a valine residue, a phenylalanine residue, a tyrosine residue, a tryptophan residue, an asparagine residue, a serine residue, a glutamine residue, a threonine residue, an aspartic acid residue, a glutamic acid residue, an arginine residue, a histidine residue, a methionine residue, a thiol group-containing amino acid residue forming a cyclized portion, or a halocarboxyl group-containing amino acid residue forming a cyclized portion,
X6 represents any amino acid residue, and
X4 represents an alanine residue, an isoleucine residue, a phenylalanine residue, a tyrosine residue, an asparagine residue, a histidine residue, or a methionine residue.

8. The cyclic peptide or a salt thereof according to claim 1,
wherein Xm represents a group represented by -X5-X6-X4-,
X5 represents an alanine residue, a proline residue, a leucine residue, an isoleucine residue, a phenylalanine residue, a tyrosine residue, a tryptophan residue, an asparagine residue, a glutamine residue, a threonine residue, an aspartic acid residue, a glutamic acid residue, an arginine residue, a histidine residue, or a methionine residue,
X6 represents any amino acid residue,
X4 represents an isoleucine residue, a phenylalanine residue, a tyrosine residue, an asparagine residue, a histidine residue, or a methionine residue, and
Xk represents a group represented by -X6-X4-.

9. The cyclic peptide or a salt thereof according to claim 4,
wherein L represents a cyclized portion represented by 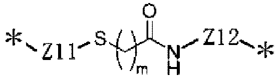 or 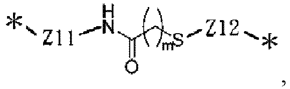 where Z11 and Z12 each independently represent a linking group, m represents an integer of 1 to 10, and * represents a position of an α-carbon atom of an amino acid main chain, and
L1 represents a cyclized portion represented by 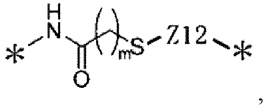 where Z12 represents a linking group, m represents an integer of 1 to 10, and * represents a position of an α-carbon atom of an amino acid main chain.

10. The cyclic peptide or a salt thereof according to claim 1,
wherein a ring is composed of 10 to 22 amino acid residues.

11. The cyclic peptide or a salt thereof according to claim 1,
wherein the cyclized portion contains a homocysteine residue.

12. The cyclic peptide or a salt thereof according to claim 1,
wherein the cyclic peptide has any one of amino acid sequences set forth in SEQ ID NOs: 37 to 450 and 452 to 454 described in Tables 4 to 6, or
the cyclic peptide has an amino acid sequence in which 1 to 4 amino acids in any one of the amino acid sequences set forth in SEQ ID NOs: 37 to 450 and 452 to 454 described in Tables 4 to 6 have been substituted, deleted, or inserted, and has a binding ability to an FGFR protein.

13. The cyclic peptide or a salt thereof according to claim 1,
wherein the cyclic peptide has any one of amino acid sequences set forth in SEQ ID NOs: 37 to 51, 53 to 55, 57 to 61, 63 to 68, 70 to 89, 92, 93, 103, 104, 106 to 109, 114, 115, 117, 119, 121 to 123, 125 to 133, 136 to 181, 187 to 189, 194 to 199, 201 to 203, 205 to 207, 211 to 218, 220 to 235, 237 to 250, 254 to 270, 272, 275, 276, 278 to 291, 293 to 324, 328 to 337, 339, 341 to 354, 356, 358 to 360, 385, 386, 391, 392, 394 to 398, 400, 402, 403, 406, 407, 409 to 411, 430, 433 to 435, 437 to 444, 446 to 450, and 452 described in Tables 4 to 6, or
the cyclic peptide has an amino acid sequence in which 1 to 4 amino acids in any one of the amino acid sequences set forth in SEQ ID NOs: 37 to 51, 53 to 55, 57 to 61, 63 to 68, 70 to 89, 92, 93, 103, 104, 106 to 109, 114, 115, 117, 119, 121 to 123, 125 to 133, 136 to 181, 187 to 189, 194 to 199, 201 to 203, 205 to 207, 211 to 218, 220 to 235, 237 to 250, 254 to 270, 272, 275, 276, 278 to 291, 293 to 324, 328 to 337, 339, 341 to 354, 356, 358 to 360, 385, 386, 391, 392, 394 to 398, 400, 402, 403, 406, 407, 409 to 411, 430, 433 to 435, 437 to 444, 446 to 450, and 452 described in Tables 4 to 6 have been substituted, deleted, or inserted, and has a binding ability to an FGFR protein.

14. The cyclic peptide or a salt thereof according to claim 1,
wherein the cyclic peptide has any one of amino acid sequences set forth in SEQ ID NOs: 37 to 42, 45, 47 to 51, 53 to 55, 60 to 61, 63, 65 to 68, 71 to 88, 92 to 93, 106, 108, 114 to 115, 117, 119, 121, 123, 125 to 133, 136 to 170, 172 to 180, 187 to 188, 194 to 199, 201, 203, 205 to 207, 211 to 212, 214 to 218, 220 to 235, 237 to 250, 254 to 260, 262 to 270, 272, 275, 278 to 290, 293, 295 to 324, 328 to 337, 339, 341 to 354, 356, 358 to 360, 391 to 392, 395, 397, 400, 402, 406, 409 to 411, 430, 433 to 435, 437 to 443, 446 to 450, and 452 described in Tables 4 to 6, or
the cyclic peptide has an amino acid sequence in which 1 to 4 amino acids in any one of the amino acid sequences set forth in SEQ ID NOs: 37 to 42, 45, 47 to 51, 53 to 55, 60 to 61, 63, 65 to 68, 71 to 88, 92 to 93, 106, 108, 114 to 115, 117, 119, 121, 123, 125 to 133, 136 to 170, 172 to 180, 187 to 188, 194 to 199, 201, 203, 205 to 207, 211 to 212, 214 to 218, 220 to 235, 237 to 250, 254 to 260, 262 to 270, 272, 275, 278 to 290, 293, 295 to 324, 328 to 337, 339, 341 to 354, 356, 358 to 360, 391 to 392, 395, 397, 400, 402, 406, 409 to 411, 430, 433 to 435, 437 to 443, 446 to 450, and 452 described in Tables 4 to 6 have been substituted, deleted, or inserted, and has a binding ability to an FGFR protein.

15. The cyclic peptide or a salt thereof according to claim 1,
wherein the cyclic peptide is modified with another substance.

16. A cyclic peptide complex or a salt thereof, in which two or more molecules of the cyclic peptide or a salt thereof according to any one of claims 1 to 15 are linked by a linker.

17. A cyclic peptide complex or a salt thereof, in which two molecules of the cyclic peptide or a salt thereof according to any one of claims 1 to 15 are linked by a linker.

18. The cyclic peptide complex or a salt thereof according to claim 16,
wherein the cyclic peptide complex is modified with another substance.

19. A culture medium composition or a culture medium additive, comprising:
the cyclic peptide or a salt thereof according to any one of claims 1 to 15.

20. A material for purification comprising:
the cyclic peptide or a salt thereof according to any one of claims 1 to 15.

21. A material for labeling comprising:
the cyclic peptide or a salt thereof according to any one of claims 1 to 15.

22. A material for cell regulation comprising:
the cyclic peptide or a salt thereof according to any one of claims 1 to 15.

23. A material for accumulation comprising:
the cyclic peptide or a salt thereof according to any one of claims 1 to 15.

24. A culture medium composition or a culture medium additive, comprising:
the cyclic peptide complex or a salt thereof according to claim 16.

25. A material for purification comprising:
the cyclic peptide complex or a salt thereof according to claim 16.

26. A material for labeling comprising:
the cyclic peptide complex or a salt thereof according to claim 16.

27. A material for cell regulation comprising:
the cyclic peptide complex or a salt thereof according to claim 16.

28. A material for accumulation comprising:
the cyclic peptide complex or a salt thereof according to claim 16.
